# EUROPÄISCHE PATENTSCHRIFT

(11) **EP 0 541 593 B1**
(45) Veröffentlichungstag und Bekanntmachung des Hinweises auf die Patenterteilung: **13.09.1995**
(21) Anmeldenummer: 91912996.5
(22) Anmeldetag: 22.07.1991
(51) Int. Cl.: C07C 405/00, A61K 31/557

(54) **CYCLOPENTENDERIVATE, VERFAHREN ZU IHRER HERSTELLUNG UND IHRE PHARMAZEUTISCHE VERWENDUNG**
CYCLOPENTANE DERIVATIVES, PROCESS FOR PRODUCING THEM AND THEIR PHARMACEUTICAL USE
DERIVES DE CYCLOPENTANE, PROCEDE POUR LEUR FABRICATION, AINSI QUE LEUR UTILISATION PHARMACEUTIQUE

(30) Priorität: 27.07.1990 DE 4024345
(43) Veröffentlichungstag der Anmeldung: 19.05.1993
(73) Patentinhaber: SCHERING AKTIENGESELLSCHAFT, 13353 Berlin (DE)
(72) Erfinder: KLAR, Ulrich, D-1000 Berlin 27 (DE); REHWINKEL, Hartmut, D-1000 Berlin 44 (DE); VORBRÜGGEN, Helmut, D-1000 Berlin 27 (DE); THIERAUCH, Karl-Heinz, D-1000 Berlin 37 (DE); VERHALLEN, Peter, D-1000 Berlin 28 (DE)
(86) Internationale Anmeldenummer: DE9100603
(87) Internationale Veröffentlichungsnummer: WO9202494

(56) Entgegenhaltungen:
- EP-A- 0 069 049
- DE-A- 3 923 798
- GB-A- 1 420 338
- GB-A- 1 539 268
- US-A- 3 933 904
- US-A- 4 025 546
- Chemical Abstracts, Band 111, Nr. 1, 3, Juli 1989, (Columbus, Ohio, US), siehe Seite 681, Zusammenfassung 7134r, & JP, A, 63277655
- Chemical Abstracts, Band 81, Nr. 21, 25 November 1974, (Columbus, Ohio, US), C. Gandolfi et al.: "Prostaglandins. VI. 9-Deoxy-9(10)-prostatrienoic acids" siehe Seite 375, Zussammenfassung 135499n.
- Chemical Abstracts, Band 85, Nr. 27, 25 October 1976, (Columbus, Ohio,US), C. Gandolfi et al.: "13-Dehydroprostaglandins", siehe Seite 65, Zussammenfassung 117046r,

## Beschreibung

Die Erfindung betrifft Cyclopenten-Derivate, Verfahren zu ihrer Herstellung sowie ihre Verwendung als Hilfsstoffe für pharmakologische Untersuchungen und als Arzneimittel. Cyclopentan-Derivate sind in den letzten Jahren intensiv bearbeitet worden, da vom Cyclopentan-System abgeleitete Prostaglandine wie z.B. PGA₂, PGB₂, PGE₂, 6-Oxo-PGE₁, PGD₂, PGF_{2α}, PGJ₂ und ihre Analoga unterschiedlichste biologische Wirkungen z.B. auf das Herz-Kreislauf-, ZNS- oder Immun-System besitzen.
Es wurde überraschenderweise gefunden, daß durch die Einführung einer Doppelbindung in Position 9 oder 10 (Prostaglandin-Zählweise) des Prostangerüstes in Kombination mit unterschiedlichsten Strukturmerkmalen in der unteren Kette sowie in Position 11 chemisch und metabolisch stabile Prostaglandin-Analoga erhalten werden, die in der Lage sind, die pharmakologischen Eigenschaften des instabilen Thromboxan-A₂ (TXA₂) bzw. PGH₂ sowie seiner stabilen Analoga wie z.B. U46619 oder U44069 am Rezeptor zu antagonisieren.
Die Verbindungen dieser Erfindung stellen deshalb wertvolle Hilfsmittel zur selektiven Therapie von Erkrankungen, die auf einen Überschuß an TXA₂ bzw. PGH₂ zurückzuführen sind, dar.

Die Erfindung betrifft Cyclopentenderivate der Formel I, 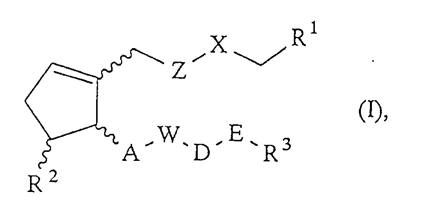
worin 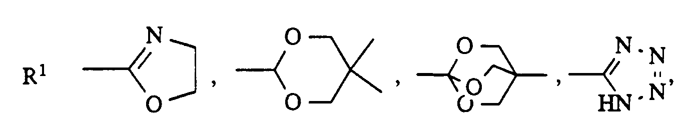
oder COOR⁴, wobei R⁴ Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y substituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR⁶ mit R⁶ in der Bedeutung Wasserstoff, C₁-C₁₀-Alkyl, C₁-C₁₀-Alkanoyl, C₆-C₁₂-Arylsulfonyl oder C₁-C₁₀-Alkansulfonyl sein kann,
X -(CH₂)ₚ-, -CH₂-O-, oder -CH₂-S-,
Z,A unabhängig voneinander eine Direktbindung, (Z)-CH=CH-, (E)-CH=CH-, oder -C≡C-,
p 0 bis 5,
n,r unabhängig voneinander 0 bis 2,
R² OR⁵ oder R⁵,
W eine Direktbindung, eine -[(CH₂)ₙ-V]_{q}-Gruppe, eine -(CH₂)ₙ-V-(CH₂)_{q}-V-Gruppe, eine freie oder funktionell abgewandelte Hydroxymethylengruppe, oder eine freie oder funktionell abgewandelte 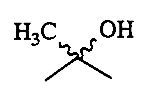
-Gruppe,
wobei die Hydroxygruppe jeweils α- oder β-ständig sein kann,
q 1 oder 2,
D eine Direktbindung, eine geradkettige gesättigte Alkylengruppe mit 1-5 C-Atomen, eine verzweigte gesättigte Alkylengruppe oder eine geradkettige oder verzweigte ungesättigte Alkylengruppe mit 2-5 C-Atomen, die gegebenenfalls durch Fluoratome substituiert sein können, - (CH₂)ₙ-NH-SO₂-, 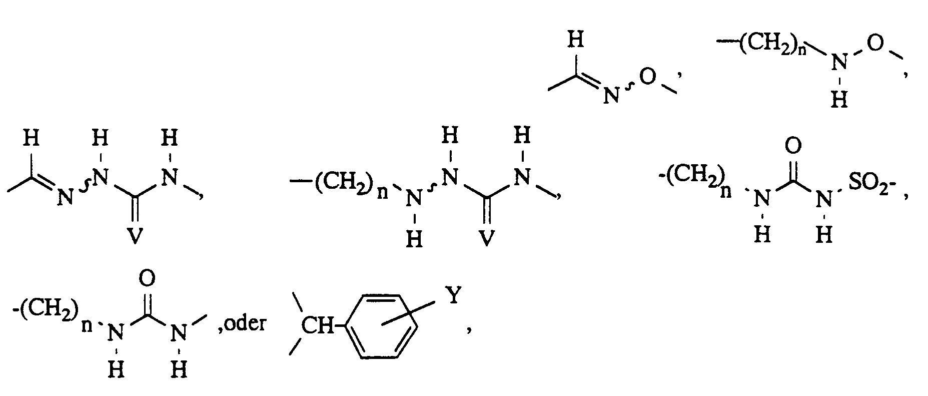
V ein O- oder S-Atom,
E eine Direktbindung, -C≡C- oder -CH=CR⁷-, wobei R⁷ Wasserstoff, C₁-C₅-Alkyl, Halogen oder Trifluormethyl,
AWDE gemeinsam eine Direktbindung,
AW gemeinsam eine Direktbindung,
DE gemeinsam eine Direktbindung sein können, 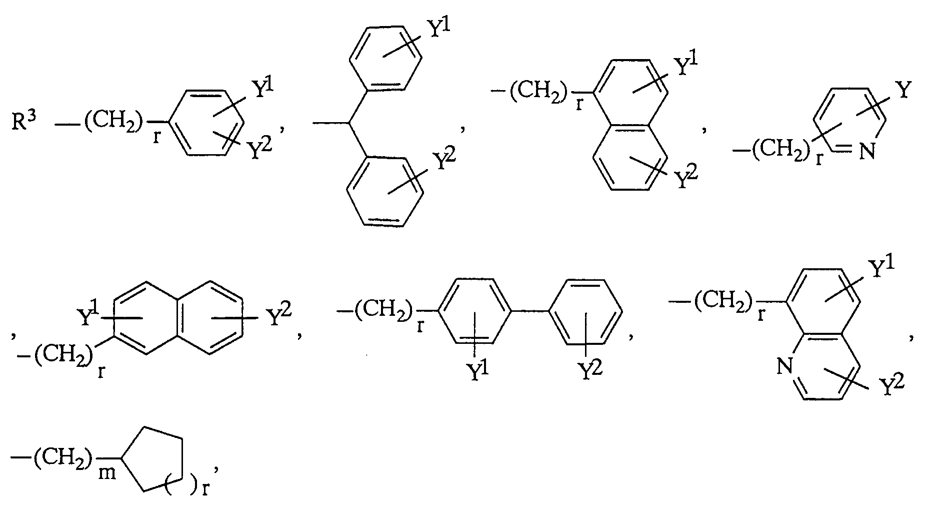
C₃-C₁₀-Cycloalkyl oder gegebenenfalls durch Y substituiertes C₁-C₁₀-Alkyl,
wobei R³ nur 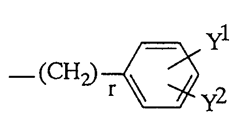
sein kann, wenn A oder W eine Direktbindung bedeuten,
m 1 oder 2,
Y¹ und Y² gleich oder verschieden sind und Y bedeuten,
Y Wasserstoff, Halogen, N₃, CF₃, OR⁵, NO₂, NH₂, CN, COOR⁵ oder C₁-C₁₀-Alkyl,
R⁵ Wasserstoff, gegebenenfalls durch Halogen substituiertes C₁-C₁₀-Alkyl, C₆-C₁₂-Aryl oder C₇-C₁₆-Aralkyl sein kann und, falls R⁴ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.
Die Definition 5- oder 6-gliedriger heterocyclischer Rest betrifft Heterocyclen, die wenigstens ein Heteroatom, vorzugsweise Stickstoff, Sauerstoff oder Schwefel enthalten und mono- oder bicyclisch sind. Beispielsweise seien genannt 2-Furyl, 3-Furyl, 2-Thienyl, 3-Thienyl, 2-Pyridyl, 3-Pyridyl, 4-Pyridyl, Chinolyl, Isochinolyl.
Als Alkylgruppen R³, R⁴, R⁵, E und Y sind gerad- oder verzweigtkettige Alkylgruppen mit 1 - 10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R³, R⁴, R⁵, E und Y können substituiert sein durch Halogenatome, Dimethylamino, Diethylamino, Methoxy, Ethoxy.
Als bevorzugte Alkylgruppen R³, R⁴, R⁵, E und Y sind solche mit 1 - 5 C-Atomen, wie z.B. Methyl, Ethyl, Propyl, Isobutyl, Butyl, Neopentyl, tert.-Butyl , Chlorethyl, 1-oder 2-Chlorpropyl, Hydroxyethyl, 1- oder 2-Hydroxypropyl zu nennen.
Als Arylgruppen R⁴ und R⁵ kommen beispielsweise in Betracht: Phenyl, Diphenyl, 1-Naphthyl und 2-Naphthyl, die substituiert sein können durch 1 - 3 Halogenatome, eine Phenylgruppe, 1 - 3 Alkylgruppen mit jeweils 1 - 4 C-Atomen eine Chlormethyl-, Fluormethyl-, Carboxyl-, C₁-C₄-Alkoxy- oder Hydroxygruppe. Bevorzugt ist die Substitution in 3- und 4-Stellung am Phenylring zum Beispiel durch Fluor, Chlor, C₁-C₄- Alkoxy oder Trifluormethyl oder in 4-Stellung durch Hydroxy.
Die Cycloalkylgruppen R⁵ können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl, Cyclohexyl, Methylcyclopentyl, Methylcyclohexyl.
Die unter den Definitionen genannten C₁-C₁₀-Alkylgruppen sollen geradkettige oder verzweigte Alkylgruppen sein, wie sie für die vorstehenden Alkylgruppen bereits genannt wurden.
Die Hydroxygruppen in R², Y und W können funktionell abgewandelt sein, beispielsweise durch Veretherung oder Veresterung, wobei die freie oder abgewandelte Hydroxygruppe in W α- oder β-ständig sein kann, wobei freie Hydroxygruppen bevorzugt sind. Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest. Als Acylreste kommen z.B. Acetyl, Propionyl, Butyryl, Benzoyl in Frage.
Halogen in den Definitionen für R⁴, R⁵, E und Y bedeutet Fluor, Chlor, Brom und Iod. Die Reste "C₁-C₁₀-Alkanoyl" oder "C₁-C₁₀-Alkansulfonyl" für R⁶ entsprechen den bereits genannten Alkylgruppen gleicher Länge mit dem Unterschied, daß sie an eine Carboxylgruppe bzw. Sulfonylgruppe gebunden sind. Bevorzugt sind C₁-C₄-Alkanoyl bzw. -Alkansulfonyl.
Zur Salzbildung mit den freien Säuren (R⁴ = H) sind anorganische und organische Basen geeignet, wie sie dem Fachmann zur Bildung physiologisch verträglicher Salze bekannt sind. Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)methylamin u.s.w.

Bevorzugt werden die Verbindungen der Formel I, in denen
- R¹: die Gruppe COOR⁴ oder CONHR⁶,
- R²: Wasserstoff, Hydroxyl, gegebenenfalls durch Halogen substituiertes C₁-C₄-Alkyl, C₆-C₁₂-Aryl oder C₇-C₁₆-Aralkyl,
- R⁴: Wasserstoff, oder gegebenenfalls durch Halogen substituiertes C₇-C₁₆-Aralkyl, C₅-C₆-Cycloalkyl oder C₁-C₁₀-Alkyl
- R⁶: C₁-C₇-Alkanoyl, C₆-C₁₂-Arylsulfonyl oder C₁-C₇-Alkansulfonyl,
- p: 0 bis 4
- n,r: unabhängig voneinander 0 oder 1 bedeuten.

Besonders bevorzugt werden die Verbindungen der Formel I, in denen
- R¹: die Gruppe COOR⁴,
- R²: Wasserstoff, Hydroxyl, Phenyl oder Phenylethyl,
- R⁴: Wasserstoff oder Methyl,
- R⁶: Methansulfonyl,
- p: 0 bis 4
- n,r: unabhängig voneinander 0 oder 1 bedeuten.

Die anmeldungsgemäßen Verbindungen der Formel I lassen sich, wie nachfolgend näher beschrieben, herstellen: 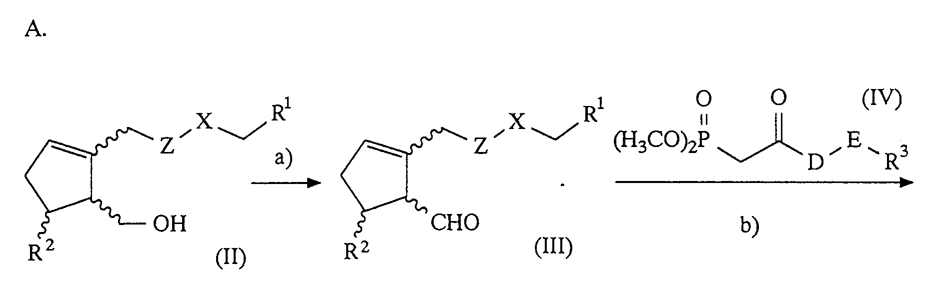 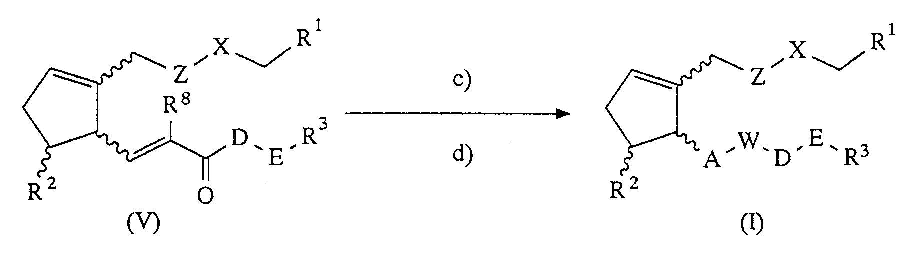
mit R², R³, A, E, X, Z in den oben angegebenen Bedeutungen,
D als gegebenenfalls durch Alkyl substituiertes Alkylen, 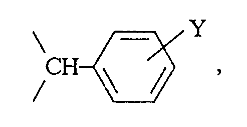
R¹ in der Bedeutung eines -COOR⁴-Esters,
R⁸ als Wasserstoff oder Brom,
W -CH(OH)-. 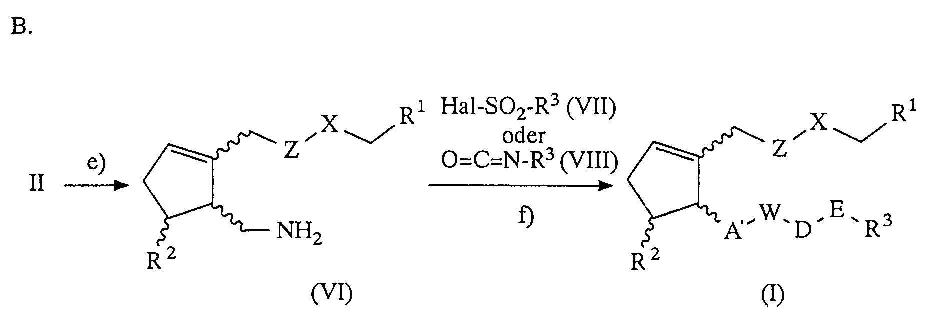
mit R², R³, X, Z, Hal in den oben angegebenen Bedeutungen,
AW, E als Direktbindung
D in der Bedeutung -(CH₂)₁-NH-SO₂-, 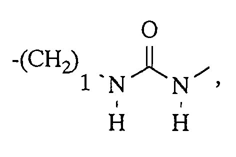
R¹ in der Bedeutung eines -COOR⁴-Esters. 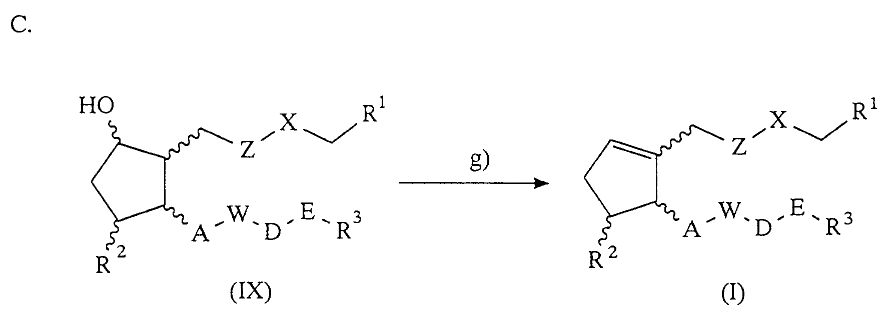
mit R², R³, X, Z, Hal in den oben angegebenen Bedeutungen,
AW, E als Direktbindung
D in der Bedeutung -(CH₂)₀-NH-SO₂-, 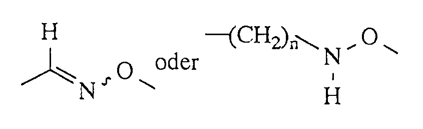
R¹ in der Bedeutung eines -COOR⁴-Esters. 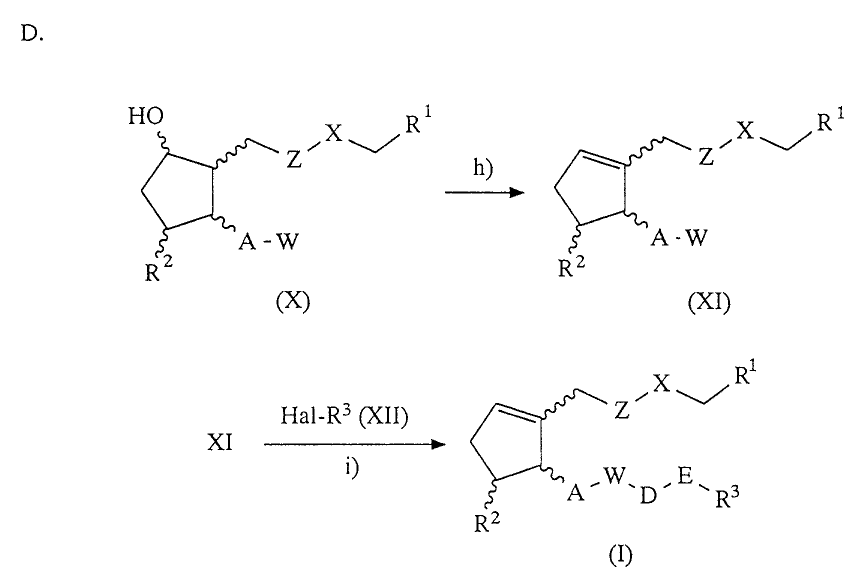
mit R², R³, n, V, X, Z, Hal in den oben angegebenen Bedeutungen,
A, DE als Direktbindung
W in der Bedeutung -[(CH₂)ₙ-V]ₙ-
R¹ in der Bedeutung eines -COOR⁴-Esters. 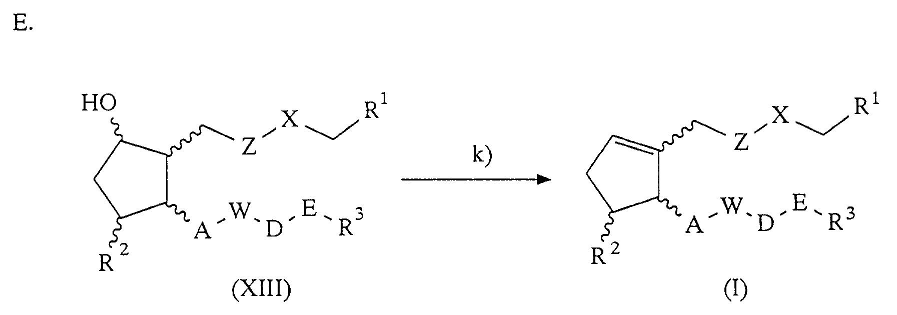
mit R², R³, n, V, W, X, Z, Hal in den oben angegebenen Bedeutungen,
A, DE als Direktbindung
W in der Bedeutung - [(CH₂)ₙ-V]ₙ-
R¹ in der Bedeutung eines -COOR⁴-Esters.

Die Verbindungen der Formel I lassen sich nach Anspruch 3 entsprechend den oben beschriebenen Verfahrensalternativen herstellen.

Die Reaktionsbedingungen der Verfahrensstufen sind:

### a) II ⇒ III (Verfahren A)

Die Oxidation von Verbindungen der Formel II erfolgt nach bekannten Verfahren, wie z.B. nach dem von Swern, Collins sowie unter Verwendung von Pyridiniumdichromat bzw. -chlorochromat in Lösungsmitteln wie Dichlormethan, Diethylether, Tetrahydrofuran, Benzol oder Toluol bei -80°C bis -50°C (Swern) bzw. bis +30°C (bei den übrigen Oxidationen) innerhalb von 10 Minuten bis 8 Stunden.

### b) III ⇒ V (Verfahren A), c),d) V ⇒ I (Verfahren A)

Die Umsetzung der Verbindungen III mit den Phosphonaten IV sowie die sich anschließende Reduktion bzw. HBr-Eliminierung erfolgen analog den in der DE-OS 2845770 genannten Bedingungen.

### e) II ⇒ VI (Verfahren B)

Die Umsetzung der Verbindungen der Formel II zu Verbindungen der Formel VI erfolgt wie in den dafür genannten Beispielen 38a bis 38c.

### f) VI ⇒ I (Verfahren B)

Die umsetzung der Verbindungen der Formel VI mit den Verbindungen der Formel VII oder VIII erfolgt wie in dem dafür genannten Beispiel 38.

### g) IX ⇒ I (Verfahren C)

Die Umsetzung der Verbindungen der Formel IX zu Verbindungen der Formel I erfolgt wie in Beispiel 1 beschrieben, wobei zuvor in D enthaltene C-C-Mehrfachbindungen gegebenenfalls nach den dem Fachmann bekannten Methoden hydriert werden.

### h) X ⇒ XI (Verfahren D)

Die Umsetzung der Verbindungen der Formel X zu Verbindungen der Formel XI erfolgt wie in Beispiel 1 beschrieben, wobei zuvor in D enthaltene C-C-Mehrfachbindungen gegebenenfalls nach den dem Fachmann bekannten Methoden hydriert werden.

### i) XI ⇒ I (Verfahren D)

Die Umsetzung der Verbindungen der Formel XI mit Verbindungen der Formel XII zu Verbindungen der Formel I erfolgt wie in Beispiel 15 beschrieben.

### k) XIII ⇒ I (Verfahren E)

Die Umsetzung der Verbindungen der Formel XIII zu Verbindungen der Formel I erfolgt wie in Beispiel 1 beschrieben, wobei zuvor in D enthaltene C-C-Mehrfachbindungen gegebenenfalls nach den dem Fachmann bekannten Methoden hydriert werden.

Die Freisetzung der funktionell abgewandelten Hydroxygruppen R², R³ und W erfolgt nach den dem Fachmann bekannten Methoden. Beispielsweise wird die Abspaltung von Etherschutzgruppen in einer wässrigen Lösung einer organischen Säure, wie z.B. Essigsäure, Propionsäure, Zitronensäure u.a. oder in einer wässrigen Lösung einer anorganischen Säure, wie z.B. Salzsäure, oder im Falle von Tetrahydropyranylethern unter Verwendung von Pyridinium-p-Toluolsulfonat, vorzugsweise in Alkoholen als Lösungsmittel oder unter Verwendung von wasserfreiem Magnesiumbromid, vorzugsweise in Diethylether als Lösungsmittel durchgeführt.
Zur Verbesserung der Löslichkeit wird bei Verwendung wässrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofuran bevorzugt angewendet wird.

Die Abspaltung der Silyletherschutzgruppen erfolgt beispielsweise mit Tetrabutylammoniumfluorid nach den dem Fachmann bekannten Methoden. Als Lösungsmittel sind beispielsweise Tetrahydrofuran, Diethylether, Dioxan, Methylenchlorid etc. geeignet. Die Abspaltung wird vorzugsweise bei Temperaturen zwischen 20°C und 80°C durchgeführt.

Die Verseifung der Acylgruppen und Ester in COOR⁴ der Cyclopentenderivate wird nach den dem Fachmann bekannten Methoden durchgeführt, wie beispielsweise mit basischen Katalysatoren wie z.B. mit Alkali- oder Erdalkali-carbonaten oder -hydroxiden in einem Alkohol oder der wässrigen Lösung eines Alkohols. Als Alkohole kommen aliphatische Alkohole wie z.B. Methanol, Ethanol, Butanol etc. in Betracht, vorzugsweise jedoch Methanol. Als Alkalicarbonate und -hydroxide seien Lithium-, Natrium- und Kaliumsalze genannt. Bevorzugt sind die Lithium- und Kaliumsalze. Als Erdalkalicarbonate und -hydroxide eignen sich beispielsweise Calciumcarbonat, Calciumhydroxid und Bariumcarbonat. Die Umsetzung erfolgt allgemein bei -10°C bis +70°C, vorzugsweise jedoch bei +25°C.

Die Einführung der Estergruppe CO₂R⁴ für R¹ bzw. CO₂R⁵ für Y, bei welcher R⁴ bzw. R⁵ eine Alkylgruppe mit 1-10-C-Atomen darstellt, erfolgt nach den dem Fachmann bekannten Methoden. Die Carboxyverbindungen (R⁴ = H bzw. R⁵ = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der Carboxyverbindung, gelöst in dem gleichen oder in einem anderen ebenfalls inerten Lösungsmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389-394(1954)].

Die Einführung der Estergruppe CO₂R⁴ für R¹ bzw. CO₂R⁵ für Y, bei welcher R⁴ bzw. R⁵ eine substituierte oder unsubstituierte Alylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base wie z.B. Pyridin, DMAP, Triethylamin, in einem inerten Lösungsmittel wie z.B. Methylenchlorid, Ethylenchlorid, Chloroform, Essigsäureethylester, Tetrahydrofuran, vorzugsweise jedoch mit Chloroform umgesetzt. Die Reaktion wird bei Temperaturen zwischen -30°C und +50°C, vorzugsweise bei +10°C, durchgeführt.

Die Cyclopentenderivate der Formel I mit R⁴ bzw. R⁵ in der Bedeutung eines Wasserstoffatomes können mit geeigneten Mengen der entsprechenden anorganischen Basen unter Neutralisierung in Salze überführt werden. Beispielsweise erhält man beim Lösen der entsprechenden Säuren in Wasser, welches stöchiometrische Mengen der Base enthält, nach Abdampfen des Wassers oder nach Zugabe eines mit Wasser mischbaren Lösungsmittels, z.B. Alkohol oder Aceton, das feste anorganische Salz.

Die Herstellung der Aminsalze erfolgt in üblicher Weise. Dazu löst man die Säure in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Etherschutzgruppen wird beispielsweise mit Dihydropyran oder Methylvinylether in Methylenchlorid oder Chloroform unter Verwendung katalytischer Mengen eines sauren Kondensationsmittels wie z.B. p-Toluolsulfonsäure, umgesetzt. Der jeweilige Enolether wird im Überschuß, vorzugsweise in der 1,2- bis 10-fachen Menge des theoretischen Bedarfs, zugesetzt. Die Umsetzung erfolgt normalerweise bei -10°C bis +30°C und ist nach 2 bis 45 Minuten beendet.

Zur Einführung von Silyletherschutzgruppen wird beispielsweise mit t-Butyl-diphenylchlorsilan oder t-Butyl-dimethylchlorsilan in Dimethylformamid unter Verwendung einer Base wie z.B. Imidazol, umgesetzt. Das jeweilige Silylchlorid wird im Überschuß, vorzugsweise in der 1,05- bis 4-fachen Menge des theoretischen Bedarfs, zugesetzt. Die Umsetzung erfolgt normalerweise bei 0°C bis 30°C und ist nach 1 bis 24 Stunden beendet.

Die Einführung der Acylschutzgruppen erfolgt, indem man eine Verbindung der Formel I in an sich bekannter Weise mit einem Carbonsäurederivat, wie z.B. Säurechlorid, Säureanhydrid etc., umsetzt.

Cyclodextrinclathrate werden analog der Vorschrift in WO 87/05294 erhalten.

Liposomen werden nach dem in "Pharmazie in unserer Zeit 11, 98(1982)" beschriebenen Herstellungsverfahren hergestellt.

Alle stereoisomeren Formen gehören ebenfalls zum Gegenstand der Erfindung.

### Biologische Wirkung und Anwendungsbereich der neuen TXA₂-Antagonisten:

Die Verbindungen dieser Erfindung eignen sich zur Therapie von Erkrankungen des cardiovaskulären Systems, des Magens, des Pankreas, der Leber und der Niere. Sie wirken blutdrucksenkend und bronchodilatorisch. Sie sind hervorragend geeignet zur Hemmung der Thrombozytenaktivierung. Folglich stellen die neuen TXA₂-Antagonisten der Formel I wertvolle pharmazeutische Wirkstoffe dar. Darüber hinaus zeichnen sich die Verbindungen durch höhere Selektivität, eine wesentlich längere Wirksamkeit und eine größere Stabilität verglichen mit ähnlichen TXA₂-Antagonisten aus.
Die neuen TXA₂-Antagonisten besitzen die für diese Verbindungsklasse typischen Eigenschaften, wie z.B. Senkung des peripheren arteriellen, des koronaren und des pulmonalen Gefäßwiderstandes, Senkung des pulmonalen Blutdrucks, Senkung des systemischen Blutdrucks ohne zugleich Schlagvolumen und koronare Durchblutung zu senken, Förderung der Nierendurchblutung und der Durchblutung anderer peripherer Organe, Erhöhung der cerebralen Durchblutung, Inhibierung der Thrombozytenaktivierung und Auflösung von Thromben, Inhibierung der Bronchokonstriktion, Inhibierung der Magensäuresekretion, Zytoprotektion des Herzens, der Magen- und Darmschleimhaut, der Leber, Zytoprotektion im Pankreas und in der Niere sowie antiallergische Eigenschaften. Daher sind die neuen TXA₂-Antagonisten prinzipiell geeignet zur Behandlung des Schlaganfalles, der Prophylaxe und Therapie koronarer Herzerkrankungen, zum Beispiel der Koronarthrombose, zur Behandlung des Herzinfarktes, peripherer Arterienerkrankungen, zur Prophylaxe und Therapie bei anderen thromboembolischen Erkrankungen und bei Arteriosklerose, bei ischämischen Attacken des ZNS-Systems und anderer Durchblutungsstörungen des Gehirns wie z.B. der Migräne, zur Behandlung der Hypertonie und zur Behandlung von Krankheiten, die mit einer Erhöhung des pulmonalen Gefäßwiderstandes einhergehen wie z.B. der pulmonalen Hypertonie und zur Therapie des Schocks, des Asthmas und der allergischen Rhinitis. Sie können ferner eingesetzt werden, zur Inhibierung von Geburtswehen und zur Behandlung von Schwangerschaftstoxikosen.
Die neuen TXA₂-Antagonisten können außerdem Anwendung finden zur Verbesserung der Organfunktion nach Transplantation, zum Beispiel bei der Nierentransplantation, zur Verhinderung von Abstoßungsreaktionen, an Stelle von Heparin oder als Adjuvans bei der Dialyse oder Hämofiltration und bei der Konservierung von Blutplasmakonserven, zum Beispiel von Blutplättchenkonserven.
Die neuen TXA₂-Antagonisten besitzen eine antimetastatische Wirkung und antiproliferative Eigenschaften. Sie sind prinzipiell geeignet zur Behandlung von Neoplasien. Die neuen TXA₂-Antagonisten können in Kombination, zum Beispiel mit Carbacyclinen, Prostacyclin und seinen Analoga, 7-Oxo-prostacyclinen, Prostaglandinen und deren Derivaten und 6-Oxo-PGE₁- und 6-Oxo-9-Fluor-Prostaglandin-Derivaten, mit TXA₂-Synthetase-Inhibitoren, mit Phosphodiesterase-Inhibitoren, mit Antagonisten und Rezeptorantagonisten verschiedener Thrombozytenstimulatoren (z.B. ADP, Thrombin, Collagen, PAF, Adrenalin, Serotonin, Fibrinogen), mit Calciumantagonisten, mit Fibrinolytika und Thrombolytika, z.B. t-PA, Streptokinase, mit Heparin und anderen Antikoagulanzien, mit Cyclooxygenasehemmern, z.B. Acetylsalicylsäure, mit Hemmstoffen der Lipoxygenasen sowie Antagonisten von Lipoxygenaseprodukten, mit Vasodilatatoren wie z.B. Nitroverbindungen, mit Antihypertensiva wie z.B. β-Blockern oder mit Diuretika Verwendung finden.
Die Dosis der Verbindungen ist 0,1-1000 mg/Tag, bevorzugt 0,1-500 mg/Tag auch in mehreren Teildosierungen, wenn sie am menschlichen Patienten verabreicht werden. Die Einheitsdosis für den pharmazeutisch akzeptablen Träger beträgt 0,1-100 mg. Für die parenterale Verabreichung werden sterile, injizierbare wässrige oder ölige Lösungen benutzt. Für die orale Applikation sind beispielsweise Tabletten, Dragees oder Kapseln geeignet.
Die Erfindung betrifft damit auch Arzneimittel auf Basis der Verbindungen der allgemeinen Formel I und üblicher Hilfs- und Trägerstoffe.
Die erfindungsgemäßen Wirkstoffe sollen in Verbindung mit den in der Galenik bekannten und üblichen Hilfsstoffen z.B. zur Herstellung von Blutdrucksenkern dienen.
Der Einheitsdosisberich für die Ampulle ist 0,1-100 mg, für die Tablette 0,1-100 mg.

### Beispiel 1:

### 7-[5(S)-(4-Toluolsulfonylamino)-cyclopent-1-enyl]-5(Z)-heptensäuremethylester:

55 mg (139 µmol) der nach Beispiel 1a dargestellten Verbindung löst man in 2,5 ml wasserfreiem Toluol, versetzt mit 56 µl wasserfreiem Pyridin, kühlt unter einer Atmosphäre aus trockenem Argon auf -60°C und versetzt mit 42 µl Diethylaminoschwefeltrifluorid. Man laßt innerhalb 3,5 Stunden auf 0°C erwärmen, quencht durch Zugabe von 1 ml einer gesättigten Natriumhydrogenkarbonatlösung, verdünnt mit Wasser und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an 4 analytischen Dünnschichtplatten. Als Laufmittel dient ein Gemisch aus n-Hexan und Aceton, als Elutionsmittel Diethylether. Isoliert werden 16 mg (40 µmol, 38%) 7-[(1R,2S,5R)-2-(4-Toluolsulfonylamino)-5-fluor-cyclopentyl]-5(Z)-heptensäuremethylester sowie 20 mg (53 µmol,29%) der Titelverbindung jeweils als farbloses Öl.
IR (Film): 3270, 3050, 3010, 2930, 2860, 1730, 1600, 1440, 1330, 1305, 1160, 1100, 950, 910, 815 und 670 cm⁻¹.

### Beispiel 1a:

### 7-[(1R,2S,5S)-2-(4-Toluolsulfonylamino)-5-hydroxy-cyclopentyl]-5(Z)-heptensäuremethylester:

Die Lösung von 140 mg (280 µmol) der nach Beispiel 1b dargestellten Verbindung in 1,5 mi Methanol versetzt man mit 20 mg fein pulverisiertem Kaliumkarbonat und erhitzt 1 Stunde auf 70°C. Nach dem erkalten wird mit gesättigter Zitronensäure angesäuert, mit Wasser verdünnt und mehrfach mit Dichlormethan extrahiert. Die vereinigten organischen Extrakte trocknet man über Magnesiumsulfat und reinigt den nach Lösungsmittelabzug erhaltenen Rückstand durch Chromatographie an 2 analytischen Dünnschichtplatten. Als Laufmittel dient ein Gemisch aus n-Hexan und Ethylacetat, als Elutionsmittel Diethylether. Isoliert werden 96 mg (243 µmol, 87%) der Titelverbindung als farbloses Öl.
IR (Film): 3600-3200, 3270, 2940, 2870, 1735, 1600, 1450, 1330, 1270, 1160, 1110, 1090, 1025, 815 und 665 cm⁻¹.

### Beispiel 1b:

### 7-[(1R,2S,5S)-2-(4-Toluolsulfonylamino)-5-benzoyloxy-cyclopentyl]-5(Z)-heptensäuremethylester:

Die Lösung von 340 mg (984 µmol) des nach Beispiel 1c dargestellten Amins in 30 ml wasserfreiem Dichlormethan versetzt man mit 1,52 ml Triethylamin, 483 mg p-Toluolsulfonsäurechlorid und rührt 1,5 Stunden bei 23°C unter einer Atmosphäre aus trockenem Argon. Man gießt auf eine halbkonzentrierte Natriumchloridlösung und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an ca. 70 ml feinem Kieselgel mit einem Gradientensystem aus n-Hexan und Ethylacetat. Isoliert werden 330 mg (660 µmol, 67%) der Titelverbindung als farbloses Öl.
IR (Film): 3270, 2940, 2870, 1730, 1710, 1600, 1450, 1330, 1270, 1160, 1110, 1090, 1025, 905, 815, 710 und 665 cm⁻¹.

### Beispiel 1c:

### 7-[(1R,2S,5S)-2-Amino-5-benzoyloxy-cyclopentyl]-5(Z)-heptensäuremethylester (A) und 7-[(1R,2S,5S)-2-Trifluoracetamido-5-benzoyloxy-cyclopentyl]-5(Z)-heptensäuremethylester (B):

Die nach Beispiel 1d gewonnene Lösung versetzt man mit 1,47 ml Trifluoressigsäure und erhitzt 6 Stunden zum Rückfluß. Man läßt 14 Stunden bei 23°C stehen, engt ein und reinigt den Rückstand durch Chromatographie an ca. 250 ml feinem Kieselgel mit einem Gradientensystem Bus Dichlormethan und Methanol. Isoliert werden 2,0 g (5,79 mmol, 43% bezogen auf Edukt in Beispiel 1e) der Titelverbindung A sowie 1,53 g (3,47 mmol, 26% bezogen auf Edukt in Beispiel 1e) der Titelverbindung B.
IR (CHCl₃) von A: 3500-2600, 2950, 2870, 1710, 1670, 1450, 1275, 1190, 1140 und 835 cm⁻¹.
IR (Film) von B: 3320, 2950, 2870, 1740-1690, 1600, 1550, 1450, 1435, 1270, 1210, 1180, 1110, 1070, 1025 und 710 cm⁻¹.

### Beispiel 1d:

### 7-[(1R,2S,5S)-2-Azidocarbonyl-5-benzoyloxy-cyclopentyl]-5(Z)-heptensäuremethylester:

Den nach Beispiel 1e gewonnenen Rückstand löst man in 20 ml Dichlormethan, kühlt auf 3°C, versetzt mit 10 mg Tetrabutylammoniumhydrogensulfat und der Lösung von 1,04 g Natriumazid in 3,5 ml Wasser. Man rührt 2,5 Stunden, verdünnt mit Dichlormethan, trennt die organische Phase ab und trocknet über frisch geglühtem Magnesiumsulfat. Die nach Filtration erhaltene Lösung wird sofort weiter umgesetzt.

### Beispiel 1e:

### 7-[(1R,2S,5S)-2-Chlorcarbonyl-5-benzoyloxy-cyclopentyl]-5(Z)-heptensäuremethylester:

Die Lösung von 5,0 g (13,4 mmol) der nach Beispiel 1f dargestellten Verbindung in 133 ml wasserfreiem Dichlormethan versetzt man unter Eiskühlung mit 2,13 ml frisch destilliertem Thionylchlorid und laßt 20 Stunden bei 23°C unter einer Atmosphäre aus trockenem Argon rühren. Man engt ein und setzt den erhaltenen Rückstand ohne Reinigung weiter um.

### Beispiel 1f:

### 7-[(1R,2S,5S)-2-Hydroxycarbonyl-5-benzoyloxy-cyclopentyl]-5(Z)-heptensäuremethylester:

Die Lösung von 30,2 g (83,9 mmol) des nach Beispiel 1g dargestellten Alkohols in 725 ml Aceton kühlt man auf -15°C, versetzt mit 44 ml einer standardisierten Chromschwefelsäurelösung (Jones-Reagenz), rührt 3 Stunden bei -10°C und zersetzt überschüssiges Oxidationsmittel durch Zugabe von 13 ml Isopropanol. Man verdünnt mit Wasser, extrahiert mehrfach mit Diethylether, wäscht die vereinigten organiaschen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 11 groben Kieselgel mit einem Gradientensystem aus n-Hexan und Ethylacetat. Isoliert werden 29,3 g (78,3 mmol, 93%) der Titelverbindung als farbloses Öl.
IR (Film): 3600-2500, 3060, 2950, 2860, 1740-1690, 1600, 1580, 1450, 1435, 1360, 1310, 1270, 1170, 1110, 1070, 1025 und 710 cm⁻¹.

### Beispiel 1g:

### 7-[(1R,2S,5S)-2-Hydroxymethyl-5-benzoyloxy-cyclopentyl]-5(Z)-heptensäuremethylester:

Die Lösung von 57,9 g (96,7 mmol) der nach Beispiel 1h dargestellten Verbindung in 124 ml wasserfreiem Tetrahydrofuran versetzt man mit 155 ml einer 1M Lösung von Tetrabutylammoniumfluorid in Tetrahydrofuran und rührt 17 Stunden bei 23°C unter einer Atmosphäre aus trockenem Argon. Man versetzt mit Wasser, extrahiert mehrfach mit Diethylether, wäscht die vereinigten organiaschen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 21 groben Kieselgel mit einem Gradientensystem aus n-Hexan und Ethylacetat. Isoliert werden 30,2 g (83,8 mmol, 87%) der Titelverbindung als farbloses Öl.
IR (Film): 3600-3200, 3060, 2940, 2860, 1735, 1710, 1600, 1580, 1360, 1310, 1275, 1170, 1110, 1070, 1025 und 715 cm⁻¹.

### Beispiel 1h:

### 7-[(1R,2S,5S)-2-(tert.-Butyldiphenylsilyloxymethyl)-5-benzoyloxy-cyclopentyl]-5(Z)-heptensäuremethylester:

Die Lösung von 47,9 g (96,7 mmol) der nach Beispiel 1i dargestellten Verbindung in 212 ml wasserfreiem Pyridin kühlt man unter einer Atmosphäre aus trockenem Argon auf 5°C, versetzt innerhalb 30 Minuten mit 29 ml Benzoylchlorid und rührt 1,5 Stunden bei 23°C. Man gießt auf 600 ml Eiswasser, extrahiert mehrfach mit Diethylether, wäscht die vereinigten organiaschen Extrakte mit 2n Salzsäure, Wasser und gesättigter Natriumchloridlösung und trocknet über Magnesiumsulfat. Den nach Filtration und Lösungsmittelabzug erhaltenen Rückstand reinigt man durch Chromatographie an ca. 21 feinem Kieselgel mit einem Gradientensystem aus n-Hexan und Ethylacetat. Isoliert werden 57,9 g (96,7 mmol, 100%) der Titelverbindung als farbloses Öl.
IR (Film): 3070, 3040, 3000, 2950, 2930, 2850, 1735, 1710, 1600, 1450, 1425, 1360, 1310, 1270, 1110, 820, 740 und 705 cm⁻¹.

### Beispiel 1i:

### 7-[(1R,2S,5S)-2-(tert.-Buryldiphenylsilyloxymethyl)-5-hydroxy-cyclopentyl]-5(Z)-heptensäuremethylester:

Die Lösung von 154 g des nach Beispiel 1j dargestellten Rohproduktes in 150 ml Aceton versetzt man mit 33,4 g Kaliumkarbonat, 41,2 g Methyliodid und erwärmt 6 Stunden auf 80°C. Man engt ein, nimmt in 400 ml Dichlormethan auf, wäscht mit Wasser und gesättigter Natriumchloridlösung und trocknet über Magnesiumsulfat. Den nach Filtration und Lösungsmittelabzug erhaltenen Rückstand reinigt man durch Chromatographie an ca. 21 groben Kieselgel mit einem Gradientensystem aus n-Hexan und Ethylacetat. Isoliert werden 47,9 g (96,8 mmol, 88% bezogen auf Edukt in Beispiel 1j) der Titelverbindung als farbloses Öl.
IR (Film): 3600-3200, 3070, 3040, 3000, 2940, 2920, 2850, 1735, 1585, 1460, 1425, 1240, 1165, 1110, 1005, 820, 740 und 700 cm⁻¹.

### Beispiel 1j:

### 7-[(1R,2S,5S)-2-(tert.-Butyldiphenylsilyloxymethyl)-5-hydroxy-cyclopentyl]-5(Z)-heptensäure:

Zu der Emulsion aus 99,7 g Carboxybutyltriphenylphosphoniumbromid in 256 ml wasserfreiem Tetrahydrofuran und 600 ml wasserfreiem Dimethylsulfoxid gibt man innerhalb einer Stunde portionsweise insgesamt 50g fein pulverisiertes Kalium-tert.-butanolat. Man rührt so lange, bis eine klare rote Lösung entsteht, tropft zügig die Lösung von 43,8 g (110 mmol) der nach Beispiel 1k dargestellten Verbindung in 130 ml wasserfreiem Tetrahydrofuran zu und läßt 2 Stunden bei 23°C unter einer Atmosphäre aus trockenem Argon reagieren. Man gießt in Eiswasser, stellt durch Zugabe einer gesättigten Zitronensäurelösung einen pH-Wert von 4-5 ein und extrahiert mehrfach mit Dichlormethan. Man wäscht mit Wasser und gesättigter Natriumchloridlösung, trocknet über Magnesiumsulfat, filtriert und engt ein. Den erhaltenen Rückstand setzt man ohne Reinigung weiter um.

### Beispiel 1k:

### (1S,3RS,5R,6S)-3-Hydroxy-6-(tert.-butyldiphenylsilyloxymethyl)-2-oxabicyclo[3.3.0]-octan:

Die Lösung von 45,8 g (116 mmol) der nach Beispiel 11 dargestellten Verbindung in 1,4 l wasserfreiem Toluol kühlt man unter einer Atmosphäre aus trockenem Argon auf - 70°C, tropft innerhalb einer Stunde 202 ml einer 1,2M Diisobutylaluminiumhydridlösung in Toluol zu und rührt 1 Stunde. Überschüssiges Reduktionsmittel wird durch Zugabe von 13 ml Isopropanol zersetzt. Man läßt auf 0°C erwärmen, tropft 100 ml Wasser zu und rührt so lange bei 23°C, bis sich ein feinkörniger Niederschlag gebildet hat. Man saugt ab, wäscht mit Dichlormethan nach und isoliert nach Lösungsmittelabzug 43,8 g (110 mmol, 95%) der Titelverbindung als farbloses Öl.
IR (Film): 3600-3200, 3070, 3050, 2940, 2850, 1590, 1465, 1425, 1390, 1360, 1260, 1110, 1010, 940, 820, 740 und 700 cm⁻¹.

### Beispiel 11:

### (1S,5R,6S)-3-Oxo-6-(tert.-butyldiphenylsilyloxymethyl)-2-oxabicyclo[3.3.0]octan:

Die Lösung von 67 g (119 mmol) des nach Beispiel 1m dargestellten Tosylates in 1,3 l Dimethoxyethan versetzt man mit 136 g Natriumiodid, 118 g Zinkstaub, 79 ml Wasser und erhitzt 16 Stunden unter Rückfluß. Nach dem Erkalten wird von ungelösten Rückständen abfiltriert, das Filtrat auf ca. 200 ml eingeengt, mit Wasser versetzt und mehrfach mit Diethylether extrahiert. Die vereinigten organiaschen Extrakte wäscht man mit 10%iger Natriumhydrogensulfatlösung, Wasser, gesättigter Natriumchloridlösung und trocknet über Magnesiumsulfat. Den nach Filtration und Lösungsmittelabzug erhaltenen Rückstand reinigt man durch Chromatographie an ca. 21 groben Kieselgel mit einem Gradientensystem aus n-Hexan und Ethylacetat. Isoliert werden 45,8 g (116 mmol, 98%) der Titelverbindung als farbloses Öl.
IR (Film): 3070, 3050, 2950, 2930, 2850, 1775, 1590, 1470, 1425, 1165, 1110, 1005, 820, 740 und 705 cm⁻¹.

### Beispiel 1m:

### (1S,5R,6S,7R)-3-Oxo-6-(tert.-butyldiphenylsilyloxymethyl)-7-toluolsulfonyloxy-2-oxabicyclo[3.3.0]octan:

Die Lösung von 67,3 g (164 mmol) des nach Beispiel 1n dargestellten Alkohols in 260 ml wasserfreiem Pyridin versetzt man mit 62,8 g p-Toluolsulfonsäurechlorid und rührt 27 Stunden unter einer Atmosphäre aus trockenem Argon bei 50°C. Man engt ein, versetzt mit Wasser und extrahiert mehrfach mit Dichlormethan. Man wäscht mit Wasser und gesättigter Natriumchloridlösung und trocknet über Magnesiumsulfat. Den nach Filtration und Lösungsmittelabzug erhaltenen Rückstand reinigt man durch Chromatographie an ca. 21 groben Kieselgel mit einem Gradientensystem aus n-Hexan und Ethylacetat. Isoliert werden 67 g (119 mmol, 72%) der Titelverbindung als farbloses Öl.
IR (CHCl₃): 3070, 3030, 2960, 2930, 2860, 1770, 1600, 1470, 1425, 1365, 1190, 1175, 1110, 1040, 980, 960, 895, 875, 820 und 700 cm⁻¹.

### Beispiel 1n:

### (1S,5R,6S,7R)-3-Oxo-6-(tert.-butyldiphenylsilyloxymethyl)-7-hydroxy-2-oxabicyclo[3.3.0] octan:

Die Lösung von 129 g (251 mmol) (1S,5R,6S,7R)-3-Oxo-6-(tert.-butyldiphenylsilyloxymethyl)-7-benzoyloxy-2-oxabicyclo[3.3.0]octan in 11 Methanol versetzt man mit 14,9 g Kaliumkarbonat und rührt 3 Stunden bei 23°C unter einer Atmosphäre aus trockenem Argon. Man versetzt mit Wasser, neutralisiert durch vorsichtige Zugabe von 2n Salzsäure, engt ein und extrahiert mehrfach mit Dichlormethan. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an ca. 21 feinem Kieselgel mit einem Gradientensystem aus n-Hexan und Ethylacetat. Isoliert werden 97 g (236 mmol, 94%) der Titelverbindung als farbloses Öl.
IR (Film): 3600-3200, 3070, 3050, 2940, 2960, 1770, 1590, 1430, 1240, 1170, 1115, 1075, 825, 745, 705, 615 und 510 cm⁻¹.

### Beispiel 2:

### 7-[5(S)-(4-Toluolsulfonylamino)-cyclopent-1-enyl]-5(Z)-heptensäure:

Die Lösung von 20 mg (58 µmol) der nach Beispiel 1 dargestellten Verbindung in 1 ml Methanol versetzt man mit 0,5 ml einer 5%igen Lithiumhydroxidlösung und rührt 1,5 Stunden bei 23°C. Durch Zugabe von gesättigter Zitronensäure wird angesäuert, mit Wasser verdünnt und mehrfach mit Dichlormethan extrahiert. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an einer analytischen Dünnschichtplatte. Als Laufmittel dient ein Gemisch aus Dichlormethan und Methanol, als Elutionsmittel ein Gemisch aus Chloroform und Isopropanol. Isoliert werden 12 mg (34 µmol, 59%) der Titelverbindung als farbloses Öl.
IR (Film): 3600-2400, 3270, 3050, 3010, 2930, 2860, 1710, 1600, 1440, 1330, 1305, 1160, 1095, 950, 910, 815, 665, 575 und 550 cm⁻¹.

### Beispiel 3:

### 7-[5(S)-(Benzolsulfonylamino)-cyclopent-1-enyl]-5(Z)-heptensäuremethylester:

80 mg (210 µmol) der nach Beispiel 3a dargestellten Verbindung setzt man in Analogie zu Beispiel 1 um und isoliert nach Aufarbeitung und Reinigung 23 mg (63 µmol, 30%) 7-[5(S)-(Benzolsulfonylamino)-cyclopent-1-enyl]-5(Z)-heptensäuremethylester sowie 26 mg (68 µmol, 32%) der Titelverbindung als farbloses Öl.
IR (Film): 3270, 3050, 3010, 2930, 2860, 1730, 1450, 1330, 1160, 1095, 755, 720 und 690 cm⁻¹.

### Beispiel 3a:

### 7-[(1R,2S,5S)-2-Benzolsulfonylamino-5-hydroxy-cyclopentyl]-5(Z)-heptensäuremethylester:

270 mg (556 µmol) der nach Beispiel 3b dargestellten Verbindung setzt man in Analogie zu Beispiel 1a um und isoliert nach Aufarbeitung und Reinigung 212 mg (556 µmol, 100%) der Titelverbindung als farbloses Öl.
IR (Film): 3600-3200, 3260, 3060, 2930, 2850, 1725, 1445, 1320, 1265, 1160, 1090, 1020 und 735 cm⁻¹.

### Beispiel 3b:

### 7-[(1R,2S,5S)-2-Benzolsulfonylamino-5-benzoyloxy-cyclopentyl]-5(Z)-heptensäuremethylester:

250 mg (724 µmol) der nach Beispiel 1c dargestellten Verbindung setzt man in Analogie zu Beispiel 1b mit Benzolsulfonsäurechlorid um und isoliert nach Aufarbeitung und Reinigung 270 mg (556 µmol, 77%) der Titelverbindung als farbloses Öl.
IR (Film): 3270, 3060, 3000, 2950, 2860, 1730, 1715, 1600, 1585, 1450, 1330, 1315, 1270, 1160, 1110, 990, 1025, 910, 755, 715 und 690 cm⁻¹.

### Beispiel 4:

### 7-[5(S)-(Benzolsulfonylamino)-cyclopent-1-enyl]-5(Z)-heptensäure:

23 mg (63 µmol) der nach Beispiel 3 dargestellten Verbindung verseift man in Analogie zu Beispiel 2 und isoliert nach Aufarbeitung und Reinigung 22 mg (63 µmol, 100%) der Titelverbindung als farbloses Öl.
¹H-NMR (CDCl₃): δ= 1,35-2,4(m, 10H), 2,45-2,55(m, 1H), 2,75-2,9(m, 1H), 4,25(m, 1H), 4,9(s, NH), 5,3-5,55(m, 3H), 7,45-7,6(m, 3H), 7,9(m, 2H).

### Beispiel 5:

### 7-[5(S)-(4-Fluorbenzylsulfonylamino)-cyclopent-1-enyl]-5(Z)-heptensäuremethylester:

84 mg (210 µmol) der nach Beispiel 5a dargestellten Verbindung setzt man in Analogie zu Beispiel 1 um und isoliert nach Aufarbeitung und Reinigung 26 mg (65 µmol, 31%) 7-[(1R,2S,5R)-2-(4-Fluorbenzol-sulfonylamino)-5-fluor-cyclopentyl]-5(Z)-heptensäuremethylester sowie 25 mg (66 µmol, 31%) der Titelverbindung jeweils als farbloses Öl.
IR (Film): 3270, 3050, 2930, 2860, 1730, 1600, 1440, 1330, 1225, 1160, 1100, 840, 735 und 670 cm⁻¹.

### Beispiel 5a:

### 7-[(1R,2S,5S)-2-(4-Fluorbenzolsulfonylamino)-5-hydroxy-cyclopentyl]-5(Z)-heptensäuremethylester:

240 mg (477 µmol) der nach Beispiel 5b dargestellten Verbindung setzt man in Analogie zu Beispiel 1b um und isoliert nach Aufarbeitung und Reinigung 175 mg (438 µmol, 92%) der Titelverbindung als farbloses Öl.
IR (Film): 3600-3200, 3310, 3060, 3020, 2940, 2860, 1730, 1610, 1595, 1490, 1440, 1325, 1250, 1160, 835, 790, 740 und 700 cm⁻¹.

### Beispiel 5b:

### 7-[(1R,2S,5S)-2-(4-Fluorbenzolsulfonylamino)-5-benzoyloxy-cyclopentyl]-5(Z)-heptensäuremethylester:

250 mg (724 µmol) der nach Beispiel 1c dargestellten Verbindung setzt man in Analogie zu Beispiel 1b mit 4-Fluorbenzolsulfonsäurechlorid um und isoliert nach Aufarbeitung und Reinigung 243 mg (483 µmol, 67%) der Titelverbindung als farbloses Öl.
IR (Film): 3270, 3060, 3000, 2950, 2870, 1730, 1710, 1590, 1570, 1490, 1450, 1335, 1270, 1165, 1150, 1090, 1025, 910, 715 und 670 cm⁻¹.

### Beispiel 6:

### 7-[5(S)-(4-Fluorbenzylsulfonylamino)-cyclopent-1-enyl]-5(Z)-heptensäure:

25 mg (66 µmol) der nach Beispiel 5 dargestellten Verbindung verseift man in Analogie zu Beispiel 2 und isoliert nach Aufarbeitung und Reinigung 22 mg (60 µmol, 91%) der Titelverbindung als farbloses Öl.
¹H-NMR (CDCl₃): δ= 1,35-2,4(m, 10H), 2,45-2,55(m, 1H), 2,8-2,9(m, 1H), 4,25(m, 1H), 4,98(d, NH), 5,3-5,55(m, 3H), 7,2(m, 2H), 7,9(m, 2H).

### Beispiel 7:

### 7-[5(S)-(Chinon-8-ylsulfonylamino)-cyclopent-1-enyl]-5(Z)-heptensäuremethylester:

74 mg (171 µmol) der nach Beispiel 7a dargestellten Verbindung setzt man in Analogie zu Beispiel 1 um und isoliert nach Aufarbeitung und Reinigung 19 mg (44 µmol, 25%) 7-[(1R,2S,5R)-2-(Chinon-8-yl-sulfonylamino)-5-fluor-cyclopentyl]-5(Z)-heptensäuremethylester sowie 29 mg (70 µmol, 41%) der Titelverbindung als farbloses Öl.
IR (Film): 3280, 3010, 2940, 2850, 1730, 1610, 1595, 1560, 1490, 1430, 1330, 1210, 1165, 1140, 1060, 835, 790 und 680 cm⁻¹.

### Beispiel 7a:

### 7-[(1R,2S,5S)-2-(Chinon-8-ylsulfonylamino)-5-hydroxy-cyclopentyl]-5(Z)-heptensäuremethylester:

339 mg (632 µmol) der nach Beispiel 7b dargestellten Verbindung setzt man in Analogie zu Beispiel 1a um und isoliert nach Aufarbeitung und Reinigung 259 mg (596 µmol, 94%) der Titelverbindung als farbloses Öl.
IR (Film): 3600-3200, 3280, 3060, 3000, 2940, 2860, 1730, 1665, 1610, 1595, 1565, 1490, 1435, 1325, 1215, 1165, 1145, 1090, 835 und 790 cm⁻¹.

### Beispiel 7b:

### 7-[(1R,2S,5S)-2-(Chinon-8-ylsulfonylamino)-5-benzoyloxy-cyclopentyl]-5(Z)-heptensäuremethylester:

250 mg (724 µmol) der nach Beispiel 1c dargestellten Verbindung setzt man in Analogie zu Beispiel 1b mit Chinon-8-ylsulfonsäurechlorid um und isoliert nach Aufarbeitung und Reinigung 339 mg (632 µmol, 87%) der Titelverbindung als farbloses Öl.
IR (Film): 3270, 3060, 2950, 2860, 1730, 1710, 1675, 1600, 1565, 1490, 1450, 1435, 1330, 1270, 1245, 1165, 1145, 1110, 1070, 1045, 1025, 900, 835, 790 und 715 cm⁻¹.

### Beispiel 8:

### 7-[5(S)-(Chinon-8-ylsulfonylamino)-cyclopent-1-enyl]-5(Z)-heptensäure:

29 mg (70 µmol) der nach Beispiel 7 dargestellten Verbindung verseift man in Analogie zu Beispiel 2 und isoliert nach Aufarbeitung und Reinigung 24 mg (61 µmol, 87%) der Titelverbindung als farbloses Öl.
IR (Film): 3600-2400, 3280, 3010, 2940, 2850, 1710, 1610, 1600, 1560, 1490, 1430, 1330, 1210, 1160, 1060, 835, 790 und 680 cm⁻¹.

### Beispiel 9:

### 7-[5(S)-(Benzolsulfonylamino)-cyclopent-1-enyl]-heptansäuremethylester:

109 mg (284 µmol) der nach Beispiel 9a dargestellten Verbindung setzt man in Analogie zu Beispiel 1 um und isoliert nach Aufarbeitung und Reinigung 34 mg (88 µmol, 31%) 7-[(1R,2S,5R)-2-(Benzolsulfonylamino)-5-fluor-cyclopentyl]-heptansäuremethylester sowie 42 mg (115 µmol, 40%) der Titelverbindung jeweils als farbloses Öl.
IR (Film): 3270, 3060, 2930, 2850, 1730, 1455, 1325, 1160, 1090, 755, 720 und 690 cm⁻¹.

### Beispiel 9a:

### 7-[(1R,2S,5S)-2-(Benzolsulfonylamino)-5-hydroxy-cyclopentyl]-5(Z)-heptansäuremethylester:

132 mg (346 µmol) der nach Beispiel 3a dargestellten Verbindung löst man in 5 ml Ethylacetat, versetzt mit 50 mg Pd/C (5%ig) und hyriert bei 1 atm, bis die theoretisch berechnete Menge Wasserstoff aufgenommen ist. Man filtriert, wäscht nach und engt ein. Isoliert werden 109 mg (284 µmol, 82%) der Titelverbindung als farbloses Öl.
IR (Film): 3600-3200, 3260, 2930, 2850, 1725, 1445, 1320, 1265, 1160, 1095, 1020 und 735 cm⁻¹.

### Beispiel 10:

### 7-[5(S)-(Benzolsulfonylamino)-cyclopent-1-enyl]-heptansäure:

42 mg (115 µmol) der nach Beispiel 9 dargestellten Verbindung verseift man in Analogie zu Beispiel 2 und isoliert nach Aufarbeitung und Reinigung 37 mg (105 µmol, 92%) der Titelverbindung als farbloses Öl.
¹H-NMR (CDCl₃): δ= 1,1-2,3(m, 14H), 2,35(t, 2H), 4,23(m, 1H), 4,5(d, NH), 5,48(d, 1H), 7,45-7,6(m, 3H), 7,9(m, 2H).

### Beispiel 11:

### 7-[5(S)-(4-Fluorbenzylsulfonylamino)-cyclopent-1-enyl]-heptansäuremethylester:

120 mg (299 µmol) der nach Beispiel 11a dargestellten Verbindung setzt man in Analogie zu Beispiel 1 um und isoliert nach Aufarbeitung und Reinigung 33 mg (82 µmol, 27%) 7-[(1R,2S,5R)-2-(4-Fluorbenzol-sulfonylamino)-5-fluor-cyclopentyl]-heptansäuremethylester sowie 44 mg (115 µmol, 38%) der Titelverbindung jeweils als farbloses Öl.
IR (Film): 3270, 3060, 2930, 2850, 1730, 1590, 1490, 1435, 1330, 1230, 1155, 1095, 840, 735 und 670 cm⁻¹.

### Beispiel 11a:

### 7-[(1R,2S,5S)-2-(4-Fluorbenzolsulfonylamino)-5-hydroxy-cyclopentyl]-5(Z)-heptansäuremethylester:

141 mg (355 µmol) der nach Beispiel 5a dargestellten Verbindung hydriert man in Analogie zu Beispiel 9a und isoliert nach Aufarbeitung 120 mg (300 µmol, 84%) der Titelverbindung als farbloses Öl.
IR (Film): 3270, 2930, 2850, 1730, 1660, 1600, 1445, 1320, 1260, 1160, 1095, 1020, 925, 840 und 820 cm⁻¹.

### Beispiel 12:

### 7-[5(S)-(4-Fluorbenzylsulfonylamino)-cyclopent-1-enyl]-heptansäure:

44 mg (115 µmol) der nach Beispiel 11 dargestellten Verbindung verseift man in Analogie zu Beispiel 2 und isoliert nach Aufarbeitung und Reinigung 38 mg (103 µmol, 89%) der Titelverbindung als farbloses Öl.
¹H-NMR (CDCl₃): δ= 1,18(m, 14H), 2,38(t, 2H), 4,23(m, 1H), 4,52(d, NH), 5,48(d, 1H), 7,2(t, 2H), 7,9(m, 2H).

### Beispiel 13:

### 7-[5(S)-(Chinon-8-ylsulfonylamino)-cyclopent-1-enyl]-heptansäuremethylester:

78 mg (179 µmol) der nach Beispiel 13a dargestellten Vebindung setzt man in Analogie zu Beispiel 2 um und isoliert nach Aufarbeitung und Reinigung 19 mg (43 µmol, 24%) 7-[(1R,2S,5R)-2-(Chinon-8-yl-sulfonylamino)-5-fluor-cyclopentyl]-heptansäuremethylester sowie 24 mg (57 µmol, 32%) der Titelverbindung jeweils als farbloses Öl.
IR (Film): 3280, 3040, 2930, 2850, 1730, 1610, 1595, 1560, 1490, 1430, 1330, 1165, 1145, 1065, 835, 790 und 680 cm⁻¹.

### Beispiel 13a:

### 7-[(1R,2S,5S)-2-(Chinon-8-ylsulfonylamino)-5-hydroxy-cyclopentyl]-5(Z)-heptansäuremethylester:

149 mg (344 µmol) der nach Beispiel 7a dargestellten Verbindung hydriert man in Analogie zu Beispiel 9a und isoliert nach Aufarbeitung 142 mg (327 µmol, 95%) der Titelverbindung als farbloses Öl.
IR (Film): 3600-3200, 3520, 3410, 3280, 2940, 2850, 1725, 1665, 1595, 1490, 1455, 1430, 1320, 1160, 1140, 1020, 890, 840, 790, 735 und 675 cm⁻¹.

### Beispiel 14:

### 7-[5(S)-(Chinon-8-ylsulfonylamino)-cyclopent-1-enyl]-heptansäure:

74 mg (177 µmol) der nach Beispiel 13 dargestellten Verbindung verseift man in Analogie zu Beispiel 2 und isoliert nach Aufarbeitung und Reinigung 61 mg (152 µmol, 86%) der Titelverbindung als farbloses Öl.
¹H-NMR (CDCl₃): δ= 0.98-2,2(m, 14H), 2,32(t, 2H), 4,3(m, 1H), 5,43(d, 1H), 6,14(d, NH), 7,56(dd, 1H), 7,65(t, 1H), 8,06(dd, 1H), 8,29(dd, 1H), 8,45(dd, 1H), 9,02(dd, 1H).

### Beispiel 15:

### 7-[(4RS,5S)-4-Phenyl-5-benzyloxymethyl-cyclopent-1-enyl]-5(E/Z)-heptensäure:

32 mg (102 µmol) der nach Beispiel 15a dargestellten Verbindung versetzt man mit 0,42 ml einer 50%igen KOH-Lösung, 265 µl Benzylchlorid, 4 mg Tetrabutylammoniumhydrogensulfat und rührt intensiv 18 Stunden bei 23 °C. Man gießt in Eiswasser, extahiert mehrfach mit Dichlormethan, trocknet die organische Phase über Magnesiumsulfat und entfernt überschüssiges Benzylchlorid durch Chromatographie an 4 analytischen Dünnschichtplatten. Als Laufmittel dient ein Gemisch aus n-Hexan und Ethylacetat, als Elutionsmittel Chloroform. Den erhaltenen Rückstand verseift man in Analogie zu Beispiel 2 und isoliert nach Aufarbeitung und Reinigung 11 mg (28 µmol, 28%) der Titelverbindung als farbloses Öl.
IR (Film): 3600-2400, 3060, 3020, 2920, 2850, 1710, 1600, 1495, 1455, 1405, 1240, 1100, 1070, 1030, 755, 735 und 700 cm⁻¹.

### Beispiel 15a:

### 7-[(4RS,5S)-4-Phenyl-5-hydroxymethyl-cyclopent-1-enyl]-5(E/Z)-heptensäure:

Die Lösung von 365 mg (660 µmol) der nach Beispiel 15b dargestellten Verbindung in 7,8 ml wasserfreiem Tetrahydrofuran versetzt man mit 1,59 ml einer 1M Lösung von Tetrabutylammoniumfluorid in Tetrahydrofuran und rührt 3 Stunden bei 23°C unter einer Atmosphäre aus trockenem Argon. Man versetzt mit Wasser, extrahiert mehrfach mit Diethylether, wäscht die vereinigten organiaschen Extrakte mit Wasser und gesättigter Natriumchloridlösung und trocknet über Magnesiumsulfat. Den nach Filtration und Lösungsmittelabzug erhaltenen Rückstand reinigt man durch Chromatographie an ca. 30 ml feinem Kieselgel mit einem Gradientensystem aus n-Hexan und Ethylacetat. Isoliert werden 165 mg (524 µmol, 80%) der Titelverbindung als farbloses Öl.
IR (Film): 3600-3200, 3060, 3020, 2920, 2850, 1730, 1450, 1375, 1255, 1150, 965, 760 und 700 cm⁻¹.

### Beispiel 15b:

### 7-[(4RS,5S)-4-Phenyl-5-(tert.-butyldiphenylsilyloxymethyl)-cyclopent-1-enyl]-5(E/Z)-heptensäuremethylester

742 mg (1,30 mmol) der nach Beispiel 15c dargestellten Verbindung setzt man in Analogie zu Beispiel 1 um und isoliert nach Aufarbeitung und Reinigung 294 mg (532 µmol, 41%) der Titelverbindung sowie 337 mg (588 µmol, 45%) 7-[(1R,2S,3RS,5R)-2-(tert.-Butyldiphenylsilyloxymetbyl)-3-phenyl-5-fluor-cyclopentyl]-5(E/Z)-heptensäuremethylester als farbloses Öl.
IR (Film): 3070, 3030, 2960, 2930, 2860, 1735, 1600, 1590, 1425, 1110, 820, 740 und 700 cm⁻¹.

### Beispiel 15c:

### 7-[(1R,2S,3RS,5S)-2-(tert.-Butyldiphenylsilyloxymethyl)-3-phenyl-5-hydroxy-cyclopentyl] -5(E/Z)-heptensäuremethylester (A) und 7-[(1R,2S,3RS,5R)-2-(tert.-Butyldiphenylsilyloxymethyl)-3-phenyl-5-hydroxy-cyclopentyl]-5(E/Z)-heptensäuremethylester (B):

1,97 g (3,47 mmol) der nach Beispiel 15d dargestellten Verbindung setzt man in Analogie zu Beispiel 15 um und isoliert nach Aufarbeitung und Reinigung 742 mg (1,30 mmol, 37%) der Titelverbindung A sowie 925 mg (1,62 mmol, 47%) der Titelverbindung B jeweils als farbloses Öl.
IR (Film) von A: 3600-3200, 3070, 3020, 2950, 2920, 2850, 1735, 1600, 1585, 1450, 1425, 1110, 820, 760, 740 und 700 cm⁻¹.
IR (Film) von B: 3600-3200, 3060, 3020, 2950, 2920, 2850, 1730, 1600, 1590, 1450, 1425, 1265, 1110, 1060, 820, 740 und 700 cm⁻¹.

### Beispiel 15d:

### 7-[(1R,2S,3RS)-2-(tert.-Butyldiphenylsilyloxymethyl)-3-phenyl-5-oxo-cyclopentyl]-5(E/Z)-heptensäuremethylester:

Die Lösung von 11,82 g (24,0 mmol) der nach Beispiel 15e dargestellten Verbindung in 120 ml wasserfreiem Tetrahydrofuran versetzt man mit 500 mg Cupfer(II)acetat, und kühlt unter einer Atmosphäre aus trockenem Argon auf -78°C. Man gibt 3,04 ml Trimethylchlorsilan und anschließend 12,8 ml einer 3M Lösung von Phenylmagnesiumbromid in Diethylether zu. Nach 45 Minuten gießt man in gesättigte Ammoniumchloridlösung und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an ca.200 ml feinem Kieselgel. Als Laufmittel dient ein Gradientensystem aus n-Hexan und Ethylacetat. Isoliert werden 9,78 g (17,2 mmol, 72%) der Titelverbindung als farbloses Öl.
IR (Film): 3070, 3030, 2950, 2930, 2850, 1735, 1600, 1465, 1450, 1425, 1110, 1055, 820, 780, 740 und 700 cm⁻¹.

### Beispiel 15e:

### 7-[(1R,2S)-2-(tert.-Butyldiphenylsilyloxymethyl)-5-oxo-cyclopent-3-enyl]-5(E/Z)-heptensäuremethylester:

Die Lösung von 5,84 g (11,5 mmol) der nach Beispiel 15f dargestellten Verbindung in 52 ml wasserfreiem Pyridin kühlt man unter einer Atmosphäre aus trockenem Argon auf 3°C, tropft 2,17 ml Methansulfonsäurechlorid zu und rührt noch 2 Stunden bei 3°C. Man versetzt mit Eis, verdünnt mit Wasser und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an ca. 200 ml feinen Kieselgel. Als Laufmittel dient ein Gradientensystem aus n-Hexan und Ethylacetat. Isoliert werden 4,93 g (10,0 mmol, 87%) der Titelverbindung als farbloses Öl.
IR (Film): 3070, 3040, 2950, 2930, 2850, 1735, 1705, 1585, 1425, 1110, 820, 740 und 700 cm⁻¹.

### Beispiel 15f:

### 7-[(1R,2S,3R)-2-(tert.-Butyldiphenylsilyloxymethyl)-3-(tetrahydropyran-2-yloxy)-5-oxo-cyclopentyl]-5(E/Z)-heptensäuremethylester:

11,9 g (20,0 mmol) der nach Beispiel 15g dargestellten Verbindung setzt man in Analogie zu Beispiel 1f um und isoliert nach Aufarbeitung und Reinigung 9,39 g (15,8 mmol, 79%) der Titelverbindung als farbloses Öl.
IR (Film): 3070, 3040, 2940, 2850, 1740, 1590, 1425, 1110, 1075, 1030, 970, 820, 740 und 700 cm⁻¹.

### Beispiel 15g:

### 7-[(1R,2S,3R,5S)-2-(tert.-Butyldiphenylsilyloxymethyl)-3-(tetrahydropyran-2-yloxy)-5-hydroxy-cyclopentyl]-5(E/Z)-heptensäuremethylester:

22,4 g (47,8 mmol) (1S,3RS,5R,6S,7R)-7-(Tetrahydropyran-2-yloxy)-6-(tert.-butyldiphenylsilyloxymethyl)-2-oxabicyclo[3.3.0]octan-3-ol setzt man in Analogie zu Beispiel 1j unter Verwendung von KOH-haltigem Kalium-tert.-butanolat um und isoliert nach Aufarbeitung und Veresterung mit einer etherischen Lösung von Diazomethan nach chromatographischer Reinigung an ca. 1,3 l feinem Kieselgel mit einem Gradientensystem aus n-Hexan und Ethylacetat 21,6 g (36,3 mmol, 76%) der Titelverbindung als farbloses Öl.
IR (Film): 3600-3200, 3070, 3040, 2940, 2850, 1730, 1595, 1425, 1110, 1075, 1025, 970, 820, 740 und 700 cm⁻¹.

### Beispiel 16:

### 7-[(4RS,5S)-4-Phenyl-5-[2-(4-fluorphenoxy)-ethoxymethyl]-cyclopent-1-enyl]-5(E/Z)-heptensäure:

30 mg (95 µmol) der nach Beispiel 15a dargestellten Verbindung setzt man in Analogie zu Beispiel 15 unter Verwendung von 2-Brom-(4-fluorphenoxy)-ethan um und isoliert nach Aufarbeitung und Reinigung 11 mg (25 µmol, 26%) der Titelverbindung als farbloses Öl.
IR (Film): 3600-2400, 3080, 3050, 3020, 2920, 2860, 1710, 1600, 1505, 1455, 1250, 1210, 1130, 825, 755, 745 und 700 cm⁻¹.

### Beispiel 17:

### 7-[(4RS,5S)-4-Phenyl-5-(3-methylbenzyloxymethyl)-cyclopent-1-enyl]-5(E/Z)-heptensäure:

24 mg (76 µmol) der nach Beispiel 15a dargestellten Verbindung setzt man in Analogie zu Beispiel 15 unter Verwendung von 3-Methylbenzylbromid um und isoliert nach Aufarbeitung und Reinigung 20 mg (49 µmol, 65%) der Titelverbindung als farbloses Öl.
IR (Film): 3600-2400, 3030, 2920, 2850, 1705, 1605, 1495, 1455, 1360, 1240, 1160, 1110, 1090, 970, 780, 755 und 700 cm⁻¹.

### Beispiel 18:

### 7-[(4RS,5S)-4-Phenyl-5-(4-cyanobenzyloxymethyl)-cyclopent-1-enyl]-5(E/Z)-heptensäure:

25 mg (80 µmol) der nach Beispiel 15a dargestellten Verbindung setzt man in Analogie zu Beispiel 15 unter Verwendung von 4-Cyanobenzylbromid um und isoliert nach Aufarbeitung und Reinigung 12 mg (29 µmol, 36%) der Titelverbindung als farbloses Öl.
IR (Film): 3600-2400, 3050, 3020, 2930, 2850, 2230, 1705, 1600, 1490, 1455, 1435, 1360, 1290, 1240, 1150, 1110, 1090, 970, 795, 760, 700 und 690 cm⁻¹.

### Beispiel 19:

### 7-[(4RS,5S)-4-Phenyl-5-(3,5-bis-trifluormethylbenzyloxymethyl)-cyclopent-1-enyl]-5(E/Z) -heptensäure:

26 mg (83 µmol) der nach Beispiel 15a dargestellten Verbindung setzt man in Analogie zu Beispiel 15 unter Verwendung von 3,5-Bis(trifluormethyl)benzylbromid um und isoliert nach Aufarbeitung und Reinigung 23 mg (44 µmol, 53%) der Titelverbindung als farbloses Öl.
IR (Film): 3600-2400, 3080, 3060, 3030, 2930, 2860, 1710, 1625, 1600, 1495, 1455, 1380, 1355, 1280, 1175, 1135, 970, 885, 845, 755, 700 und 680 cm⁻¹.

### Beispiel 20:

### 7-[(4RS,5S)-4-Phenyl-5-(1-naphtylmethoxymethyl)-cyclopent-1-en yl]-5(E/Z)-heptensäure:

24 mg (76 µmol) der nach Beispiel 15a dargestellten Verbindung setzt man in Analogie zu Beispiel 15 unter Verwendung von 1-Brommethylnaphtalin um und isoliert nach Aufarbeitung und Reinigung 16 mg (36 µmol, 48%) der Titelverbindung als farbloses Öl.
IR (Film): 3600-2400, 3050, 3020, 2930, 2850, 1705, 1600, 1455, 1280, 1235, 1165, 1100, 800, 790, 775, 760 und 700 cm⁻¹.

### Beispiel 21:

### 7-[(4RS,5R)-4-Phenyl-5-benzyloxymethyl-cyclopent-1-enyl]-5(Z)-heptensäure:

43 mg (137 µmol) der nach Beispiel 21a dargestellten Verbindung setzt man in Analogie zu Beispiel 15 um und isoliert nach Aufarbeitung und Reinigung 18 mg (46 µmol, 34%) der Titelverbindung als farbloses Öl.
IR (Film): 3600-2400, 3090, 3060, 3030, 2930, 2860, 1710, 1600, 1495, 1455, 1410, 1360, 1240, 1205, 1100, 1075, 1025, 755, 740 und 700 cm⁻¹.

### Beispiel 21a:

### 7-[(4RS,5R)-4-Phenyl-5-hydroxymethyl-cyclopent-1-enyl]-5(Z)-heptensäure:

2,27 g (4,11 mmol) 7-[(4RS,5R)-4-Phenyl-5-(tert.-butyldiphenylsilyloxymethyl)-cyclopent-1-enyl]-5(Z)-heptensäuremethylester, den man in Analogie zu den Beispielen 15b bis 15g und 1j bis 1n aus (1R,5S,6R,7S)-3-Oxo-6-(tert.-butyldiphenylsilyloxymethyl)-7-benzoyloxy-2-oxabicyclo[3.3.0]octan herstellt, setzt man in Analogie zu Beispiel 15a um und isoliert nach Aufarbeitung und Reinigung 1,28 g (4,07 mmol, 99%) der Titelverbindung als farbloses Öl.
IR (Film): 3600-2400, 3060, 3030, 2920, 2850, 1710, 1605, 1455, 1405, 1240, 1110, 1070, 1030, 755, 735 und 700 cm⁻¹.

### Beispiel 22:

### 7-[(4RS,5R)-4-Phenyl-5-[2-(4-fluorphenoxy)-ethoxymethyl]-cyclopent-1-enyl]-5(Z)-heptensäure:

43 mg (137 µmol) der nach Beispiel 21a dargestellten Verbindung setzt man in Analogie zu Beispiel 15 unter Verwendung von 2-Brom-(4-fluorphenoxy)-ethan um und isoliert nach Aufarbeitung und Reinigung 15 mg (34 µmol, 25%) der Titelverbindung als farbloses Öl.
IR (Film): 3600-2400, 3080, 3050, 3030, 2930, 2870, 1710, 1600, 1505, 1455, 1250, 1210, 1130, 830, 760, 745 und 700 cm⁻¹.

### Beispiel 23:

### 7-[(4RS,5R)-4-Phenyl-5-(3-methylbenzyloxymethyl)-cyclopent-1-enyl]-5(Z)-heptensäure:

43 mg (137 µmol) der nach Beispiel 21a dargestellten Verbindung setzt man in Analogie zu Beispiel 15 unter Verwendung von 3-Methylbenzylbromid um und isoliert nach Aufarbeitung und Reinigung 27 mg (67 µmol, 49%) der Titelverbindung als farbloses Öl.
IR (Film): 3600-2400, 3080, 3030, 2930, 2860, 1710, 1605, 1495, 1455, 1410, 1360, 1240, 1155, 1010, 985, 780, 760 und 700 cm⁻¹.

### Beispiel 24:

### 7-[(4RS,5R)-4-Phenyl-5-(4-cyanobenzyloxymethyl)-cyclopent-1-enyl]-5(Z)-heptensäure:

43 mg (137 µmol) der nach Beispiel 21a dargestellten Verbindung setzt man in Analogie zu Beispiel 15 unter Verwendung von 4-Cyanobenzylbromid um und isoliert nach Aufarbeitung und Reinigung 28 mg (67 µmol, 49%) der Titelverbindung als farbloses Öl.
IR (Film): 3600-2400, 3080, 3060, 3030, 2930, 2850, 2230, 1710, 1610, 1455, 1415, 1360, 1240, 1125, 1110, 820, 760 und 700 cm⁻¹.

### Beispiel 25:

### 7-[(4RS,5R)-4-Phenyl-5-(3-cyanobenzyloxymethyl)-cyclopent-1-enyl]-5(Z)-heptensäure:

43 mg (137 µmol) der nach Beispiel 21a dargestellten Verbindung setzt man in Analogie zu Beispiel 15 unter Verwendung von 3-Cyanobenzylbromid um und isoliert nach Aufarbeitung und Reinigung 33 mg (79 µmol, 58%) der Titelverbindung als farbloses Öl.
IR (Film): 3600-2400, 3080, 3060, 3030, 2930, 2850, 2230, 1710, 1600, 1490, 1455, 1435, 1410, 1360, 1240, 1150, 1115, 1090, 795, 760, 700 und 690 cm⁻¹.

### Beispiel 26:

### 7-[(4RS,5R)-4-Phenyl-5-[(3RS,4S)-3-hydroxy-4-methyl-non-1(E)-en-6-inyl]-cyclopent-1-enyl]-5(Z)-heptensäuremethylester:

Die Lösung von 206 mg (476 µmol) der nach Beispiel 26a dargestellten Verbindung löst man in 6 ml wasserfreiem Methanol, kühlt unter einer Atmosphäre aus trockenem Aregon auf -40°C und versetzt portionsweise mit insgesamt 105 mg Natriumborhydrid. Man läßt noch 15 Minuten reagieren, zersetzt überschüssiges Reduktionsmittel durch Zugabe von 180 µl Essigsäure, versetzt mit Wasser und extrahiert mehrfach mit Dichlomethan. Man wäscht mit Wasser, gesättigter Natriumchloridlösung und trocknet über Magnesiumsulfat. Den nach Filtration und Lösungsmittelabzug erhaltenen Rückstand reinigt man durch Chromatographie an ca 30 ml feinem Kieselgel unter Verwendung eines Gradientensystems aus n-Hexan und Ethylacetat. Isoliert werden 175 mg (403 µmol, 85%) der Titelverbindung als farbloses Öl.
IR (Film): 3600-3200, 3050, 3020, 2960, 2920, 2850, 1735, 1600, 1490, 1450, 1435, 1315, 1240, 1150, 1020, 970, 755 und 700 cm⁻¹.

### Beispiel 26a:

### 7-[(4RS,5R)-4-Phenyl-5-[3-oxo-4S-methyl-non-1(E)-en-6-inyl]-cyclopent-1-enyl]-5(Z)-heptensäuremethylester:

Zu der Aufschlämmung von 36,5 mg Natriumhydrid-Dispersion (55%ig) in 1,4 ml wasserfreiem Tetrahydrofuran tropft man bei -10°C unter einer Atmosphäre aus trockenem Argon die Lösung von 205 mg Dimethyl-(2-oxo-3S-methyl-oct-5-inyl)-phosphonat in 1,13 ml Tetrahydrofuran und rührt 15 Minuten. Man troft die Lösung von 273 mg (748 µmol) des nach Beispiel 26b dargestellten Aldehyds in 1,68 ml Tetrahydrofuran zu und rührt 1,5 Stunden bei 5°C. Man versetzt mit Essigsäure, und extrahiert mehrfach mit Diethylether. Die vereinigten organischen Extrakte wäscht man mit verdünnter Natriumhydrogenkarbonatlösung, trocknet über Magnesiumsulfat und reinigt den nach Lösungsmittelabzug erhaltenen Rückstand durch Chromatographie an ca. 30 ml feinem Kieselgel mit einem Gradientensystem aus n-Hexan und Ethylacetat. Isoliert werden 206 mg (476 µmol, 64%) der Titelverbindung als farbloses Öl.
IR (Film): 3060, 3030, 2970, 2930, 1730, 1690, 1665, 1625, 1490, 1450, 1430, 1360, 1170, 1040, 980, 760 und 700 cm⁻¹.

### Beispiel 26b:

### 7-[(4RS,5R)-4-Phenyl-5-formyl-cyclopent-1-enyl]-5(Z)-heptensäuremethylester:

Zu der unter einer Atmosphäre aus trockenem Argon bei -60°C vorgelegten Lösung von 190 µl frisch destilliertem Oxalylchlorid in 3,04 ml wasserfreiem Dichlormethan tropft man die Losung von 348 µl Dimethylsulfoxid in 1,22 ml Dichlormethan, läßt 15 Minuten reagieren und versetzt anschließend mit der Losung von 470 mg (1,50 mmol) des nach Beispiel 21a dargestellten Alkohols in 3 ml Dichlormethan. Man läßt 3 Stunden reagieren, versetzt mit 596 µl Triethylamin, gießt in Eiswasser und extrahiert mehrfach mit Dichlormethan. Die vereinigte organischen Extrakte trocknet man über Magnesiumsulfat. Den nach Filtration und Losungsmittelabzug erhaltenen Rückstand setzt man ohne Reinigung weiter um.

### Beispiel 27:

### 7-[(4RS,5R)-4-Phenyl-5-[(3RS,4S)-3-hydroxy-4-methyl-non-1(E)-en-6-inyl]-cyclopent-1-enyl]-5(Z)-heptensäure:

175 mg (403 µmol) der nach Beispiel 26 dargestellten Verbindung verseift man in Analogie zu Beispiel 2 und isoliert nach Aufarbeitung und Reinigung 143 mg (340 µmol, 84%) der Titelverbindung als farbloses Öl.
IR (Film): 3600-2400, 3080, 3030, 2960, 2930, 2860, 1710, 1600, 1460, 1290, 1270, 1070, 1010, 970, 760 und 700 cm⁻¹.

### Beispiel 28:

### 7-[(4RS,5R)-4-Phenyl-5[(3RS,4RS)-3-hydroxy-4-phenyl-pent-1(E)-enyl]-cyclopent-1-enyl]-5(Z)-heptensäuremethylester:

279 mg (630 µmol) der nach Beispiel 28a dargestellten Verbindung setzt man in Analogie zu Beispiel 26 um und isoliert nach Aufarbeitung und Reinigung 239 mg (537 µmol, 85%) der Titelverbindung als farbloses Öl.
IR (Film): 3600-3200, 3050, 3020, 2950, 2930, 2850, 1730, 1600, 1490, 1450, 1260, 1150, 1010, 970, 755, 740 und 700 cm⁻¹.

### Beispiel 28a:

### 7-[(4RS,5R)-4-Phenyl-5-[(4RS)-3-oxo-4-phenyl-pent-1(E)-enyl]-cyclopent-1-enyl]-5(Z)-heptensäuremethylester:

273 mg (748 µmol) der nach Beispiel 26b dargestellten Verbindung setzt man in Analogie Zu Beispiel 26a unter Verwendung von Dimethyl-(2-oxo-3-phenyl-butyl)-phosphonat um und isoliert nach Aufarbeitung und Reinigung 279 mg (630 µmol, 84%) der Titelverbindung als farbloses Öl.
IR (Film): 3060, 3020, 2950, 2930, 2850, 1730, 1710, 1690, 1615, 1490, 1450, 1260, 1160, 1070, 1025, 980, 755, 735 und 700 cm⁻¹.

### Beispiel 29:

### 7-[(4RS,5R)-4-Phenyl-5-[(3RS,4RS)-3-hydroxy-4-phenyl-pent-1(E)-enyl]-cyclopent-1-enyl]-5(Z)-heptensäure:

239 mg (538 µmol) der nach Beispiel 28 dargestellten Verbindung verseift man in Analogie zu Beispiel 2 und isoliert nach Aufarbeitung und Reinigung 175 mg (406 µmol, 76%) der Titelverbindung als farbloses Öl.
IR (Film): 3600-2400, 3080, 3060, 3030, 2960, 2930, 2860, 1710, 1670, 1600, 1500, 1450, 1240, 980, 760 und 700 cm⁻¹.

### Beispiel 30:

### 7-[(4RS,5R)-4-Phenyl-5-[3(RS)-hydroxy-oct-1(E)-enyl]-cyclopent-1-enyl]-5(Z)-heptensäuremethylester:

174 mg (426 µmol) der nach Beispiel 30a dargestellten Verbindung setzt man in Analogie zu Beispiel 26 um und isoliert nach Aufarbeitung und Reinigung 173 mg (421 µmol, 99%) der Titelverbindung als farbloses Öl.
IR (Film): 3600-3200, 3030, 2920, 2850, 1735, 1455, 1260, 1150, 1025, 970, 740 und 700 cm⁻¹.

### Beispiel 30a:

### 7-[(4RS,5R)-4-Phenyl-5-[3-oxo-oct-1(E)-enyl]-cyclopent-1-enyl]-5(Z)-heptensäuremethylester:

245 mg (784 µmol) der nach Beispiel 26b dargestellten Verbindung setzt man in Analogie zu Beispiel 26a unter Verwendung von Dimethyl-(2-oxo-heptyl)-phosphonat um und isoliert nach Aufarbeitung und Reinigung 174 mg (426 µmol, 54%) der Titelverbindung als farbloses Öl.
IR (Film): 3030, 2950, 2930, 2850, 1735, 1690, 1670, 1620, 1450, 1430, 1365, 1240, 1160, 1025, 860, 755 und 700 cm⁻¹.

### Beispiel 31:

### 7-[(4RS,5R)-4-Phenyl-5-[3(RS)-hydroxy-oct-1(E)-enyl]-cyclopent-1-enyl]-5(Z)-heptensäure:

173 mg (421 µmol) der nach Beispiel 30 dargestellten Verbindung verseift man in Analogie zu Beispiel 2 und isoliert nach Aufarbeitung und Reinigung 100 mg (252 µmol, 60%) der Titelverbindung als farbloses Öl.
IR (Film): 3600-3200, 3060, 3030, 2940, 2860, 1710, 1605, 1455, 1410, 1240, 970, 760 und 700 cm⁻¹.

### Beispiel 32:

### 7-[(4S,5R)-4-Hydroxy-5-[(E/Z)-diphenylmethoxyiminomethyl]-cyclopent-1-enyl]-5(Z)-heptensäuremethylester:

46 mg (89 µmol) der nach Beispiel 32a dargestellten Verbindung versetzt man mit 2 ml eines Gemisches aus Essigsäure, Wasser und Tetrahydrofuran (65:35:10) und rührt 18 Stunden bei 23°C. Man engt ein, entfernt restliche Essigsäure durch wiederholte azeotrope Destillation mit Toluol und reinigt den Rückstand durch Chromatographie an ca. 10 ml feinem Kieselgel. Isoliert werden 33 mg (76 µmol, 86%) der Titelverbindung als farbloses Öl.
IR (Film): 3600-3200, 3060, 3030, 3010, 2930, 2860, 1735, 1600, 1495, 1455, 1240, 1080, 1030, 1020, 935, 740 und 700 cm⁻¹.

### Beispiel 32a:

### 7-[(1S,2R,3S,5S)-2-[(E/Z)-Diphenylmethoxyiminomethyl]-3-(tetrahydropyran-2-yloxy)-5-fluor-cyclopentyl]-5(Z)-heptensäuremethylester (A) und 7-[(5R,4S)-5-[(E/Z)-Diphenylmethoxyiminomethyl]-4-(tetrahydropyran-2-yloxy)-cyclopent-1-enyl]-5(Z-heptensäuremethylester:

252 mg (471 µmol) der nach Beispiel 32b dargestellten Verbindung setzt man in Analogie zu Beispiel 1 um und isoliert nach Aufarbeitung und Reinigung 72 mg (134 µmol, 28%) der Titelverbindung A sowie 58 mg (112 µmol, 24%) der Titelverbindung B jeweils als farbloses Öl.
IR (Film): 3600-3200, 3060, 3030, 3010, 2940, 2870, 1730, 1600, 1455, 1245, 1020, 935, 870, 820, 745 und 700 cm⁻¹.

### Beispiel 32b:

### 7-[(1S,2R,3S,5R)-2-[(E/Z)-Diphenylmethoxyiminomethyl]-3-(tetrahydropyran-2-yloxy)-5-hydroxy-cyclopentyl]-5(Z)-heptensäuremethylester:

Die Lösung von 257 mg (493 µmol) der nach Beispiel 32c dargestellten Verbindung in 10 mi Dichlormethan versetzt man mit der etherischen Lösung von Diazomethan und engt nach beendeter Umsetzung ein. Isoliert werden 262 mg (489 µmol, 99%) der Titelverbindung als farbloses Öl.
IR (Film): 3600-3200, 3070, 3030, 3010, 2950, 2870, 1740, 1455, 1440, 1350, 1200, 1130, 1080, 1025, 975, 910, 870, 815, 745 und 705 cm⁻¹.

### Beispiel 32c:

### 7-[(1S,2R,3S,5R)-2-[(E/Z)-Diphenylmethoxyiminomethyl]-3-(tetrahydropyran-2-yloxy)-5-hydroxy-cyclopentyl]-5(Z)-heptensäure:

569 mg (889 µmol) der nach Beispiel 32d dargestellten Verbindung verseift man in Analogie zu Beispiel 2 und isoliert nach Aufarbeitung und Reinigung 399 mg (765 µmol, 86%) der Titelverbindung als farbloses Öl.
IR (Film): 3600-2400, 3070, 3030, 3010, 2940, 270, 1730, 1710, 1605, 1495, 1445, 1345, 1245, 1205, 1185, 1130, 1080, 1020, 975, 920, 870, 810, 745 und 705 cm⁻¹.

### Beispiel 32d:

### 7-[(1S,2R,3S,5R)-2-[(E/Z)-Diphenylmethoxyiminomethyl]-3-(tetrahydropyran-2-yloxy)-5-benzoyloxy-cyclopentyl]-5(Z)-heptensäuremethylester:

Die farblose Lösung von 300 mg (654 µmol) der nach Beispiel 32e dargestellten Verbindung in 16 ml wasserfreiem Ethanol versetzt man mit 120 µl wasserfreiem Pyridin, 176 mg Diphenylmethoxyamin und erhitzt 3,5 Stunden unter einer Atmosphäre aus trockenem Argon auf 50°C. Man engt ein, nimmt den Rückstand in Dichlormethan auf, wäscht mit Wasser und gesättigter Natriumchloridlösung und reinigt den nach Trocknen über Magnesiumsulfat, Filtration und Einengen erhaltenen Rückstand durch Chromatographie an ca. 50 ml Kieselgel mit einem n-Hexan/Ethylacetat-Gemisch. Isoliert werden 268 mg (453 µmol, 69%) der Titelverbindung als farbloses Öl.
IR (Film): 3380, 3210, 3100, 3010, 2950, 2870, 1735, 1715, 1690, 1600, 1535, 1450, 1360, 1320, 1275, 1115, 1070, 1030, 970, 870, 815, 760, 715 und 695 cm⁻¹.

### Beispiel 32e:

### 7-[(1S,2R,3S,5R)-2-Formyl-3-(tetrahydropyran-2-yloxy)-5-benzoyloxy-cyclopentyl]-5(Z)-heptensäuremethylester:

1,2 g (2,61 mmol) der nach Beispiel 32f dargestellten Verbindung setzt man in Analogie zu Beispiel 26b um und isoliert nach Aufarbeitung 1,2 g (2,61 mmol, 100%) der Titelverbindung, die man ohne Reinigung weiter umsetzt.

### Beispiel 32f:

### 7-[(1S,2R,3S,5R)-2-Hydroxymethyl-3-(tetrahydropyran-2-yloxy)-5-benzoyloxy-cyclopentyl]-5(Z)-heptensäuremethylester:

2,16 g (3,09 mmol) der nach Beispiel 32g dargestellten Verbindung setzt man in Analogie zu Beispiel 1g um und isoliert nach Aufarbeitung und Reinigung 1,2 g (2,61 mmol, 85%) der Titelverbindung als farbloses Öl.
IR (Film): 3600-3200, 3060, 3000, 2940, 2860, 1735, 1710, 1600, 1585, 1450, 1355, 1310, 1270, 1110, 1070, 1025, 865, 810 und 710 cm⁻¹.

### Beispiel 32g:

### 7-[(1S,2R,3S,5R)-2-(tert.-Butyldiphenylsilyloxymethyl)-3-(tetrahydropyran-2-yloxy)-5-benzoyloxy-cyclopentyl]-5(Z)-heptensäuremethylester:

59,4 g (99,9 mmol) 7-[(1S,2R,3S,5R)-2-(tert.-Butyldiphenylsilyloxymethyl)-3-(tetrahydropyran-2-yloxy)-5-hydroxy-cyclopentyl]-5(Z)-heptensäuremethylester setzt man in Analogie zu Beispiel 1h um und isoliert nach Aufarbeitung und Reinigung 62,5 g (89,4 mmol, 90%) der Titelverbindung als farbloses Öl.
IR (Film): 3070, 3050, 3010, 2950, 2860, 1740, 1715, 1600, 1585, 1450, 1425, 1275, 1110, 1025, 970, 870, 825, 790, 710, 690, 610 und 500 cm⁻¹.

### Beispiel 33:

### 7-[(4S,5R)-4-Hydroxy-5-[(E/Z)-diphenylmethoxyiminomethyl]-cyclopent-1-enyl]-5(Z)-heptensäure:

25 mg (58 µmol) der nach Beispiel 32 dargestellten Verbindung verseift man in Analogie zu Beispiel 2 und isoliert nach Aufarbeitung und Reinigung 19,5 mg (46 µmol, 80%) der Titelverbindung als farbloses Öl.
IR (Film): 3600-2400, 3090, 3060, 3030, 3010, 2930, 2860, 1710, 1600, 1495, 1455, 1240, 1080, 1030, 1020, 935, 745 und 700 cm⁻¹.

### Beispiel 34:

### 7-[(4S,5R)-4-Hydroxy-5-(3-methylbenzyloxymethyl)-cyclopent-1-enyl]-5(Z)-heptensäuremethylester:

212 mg (478 µmol) der nach Beispiel 34a dargestellten Verbindung B setzt man in Analogie zu Beispiel 32 um und isoliert nach Aufarbeitung und Reinigung 92 mg (257 µmol, 54%) der Titelver-bindung als farbloses Öl.
IR (Film): 3600-3200, 3010, 2950, 2920, 2860, 1735, 1610, 1590, 1440, 1360, 1245, 1155, 1085, 1035, 780, 740 und 695 cm⁻¹.

### Beispiel 34a:

### 7-[(1S,2R,3S,5S)-2-(3-Methylbenzyloxymethyl)-3-(tetrahydropyran-2-yloxy)-5-fluorcyclopentyl]-5(Z)-heptensäuremethylester (A) und 7-[(5R,4S)-5-(3-Methylbenzyloxymethyl)-4-(tetrahydropyran-2-yloxy)-cyclopent-1-enyl]-5(Z)-heptensäuremethylester (B):

800 mg (1,74 mmol) der nach Beispiel 34b dargestellten Verbindung setzt man in Analogie zu Beispiel 1 um und isoliert nach Aufarbeitung und Reinigung 201 mg (436 µmol, 25%) der Titelverbindung A sowie 212 mg (480 µmol, 28%) der Titelverbindung B jeweils als farbloses Öl.
IR (Film): 3600-3200, 3010, 2940, 2850, 1730, 1610, 1440, 1360, 1250, 1225, 1155, 1095, 870, 815, 780, 745 und 695 cm⁻¹.

### Beispiel 34b:

### 7-[(1S,2R,3S,5R)-2-(3-Methylbenzyloxymethyl)-3-(tetrahydropyran-2-yloxy)-5-hydroxycyclopentyl]-5(Z)-heptensäuremethylester:

1,23 g (2,75 mmol) der nach Beispiel 32f dargestellten Verbindung setzt man in Analogie zu Beispiel 15 um und isoliert nach Aufarbeitung und Reinigung 1,18 g (2,56 mmol, 93%) der Titelverbindung als farbloses Öl.
IR (Film): 3600-3300, 3010, 2940, 2860, 1740, 1610, 1595, 1440, 1355, 1250, 1200, 1155, 1115, 1075, 1020, 975, 905, 870, 815, 780, 745 und 695 cm⁻¹.

### Beispiel 35:

### 7-[(4S,5R)-4-Hydroxy-5-(3-methylbenzyloxymethyl)-cyclopent-1-enyl]-5(Z)-heptensäure:

34 mg (95 µmol) der nach Beispiel 34 dargestellten Verbindung verseift man in Analogie zu Beispiel 2 und isoliert nach Aufarbeitung und Reinigung 17 mg (49 µmol, 52%) der Titelverbindung als farbloses Öl.
IR (Film): 3600-2400, 3010, 2930, 2860, 1710, 1610, 1590, 1455, 1405, 1355, 1240, 1155, 1080, 780, 745 und 695 cm⁻¹.

### Beispiel 36:

### 7-[5R-(3-Methylbenzyloxymethyl)-cyclopent-1-enyl]-5(Z)-heptensäuremethylester:

Die Lösung von 61 mg (115 µmol) des nach Beispiel 36a dargestellten Tosylates in 0,9 ml Dimethoxyethan versetzt man mit 89,5 mg Natriumiodid, 77 mg Zinkstaub, 670 µl Wasser und erhitzt 3,5 Stunden unter Rückfluß. Nach dem Erkalten wird von ungelösten Rückständen abfiltriert, das Filtrat mit Diethylether verdünnt, mit Wasser versetzt und mehrfach mit Diethylether extrahiert. Die vereinigten organischen Extrakte wäscht man mit 10%iger Natriumhydrogensulfatlösung, Wasser, gesättigter Natriumchloridlösung und trocknet über Magnesiumsulfat. Den nach Filtration und Lösungsmittelabzug erhaltenen Rückstand reinigt man durch Chromatographie an ca. 20 ml Kieselgel mit einem Gradientensystem aus n-Hexan und Ethylacetat. Isoliert werden 32,9 mg (91 µmol, 79%) der Titelverbindung als farbloses Öl.
IR (Film): 3010, 2950, 2860, 1740, 1610, 1590, 1440, 1360, 1245, 1220, 1160, 1105, 1090, 780, 745 und 695 cm⁻¹.

### Beispiel 36a:

### 7-[(4S,5R)-4-Toluolsulfonyloxy-5-(3-methylbenzyloxymethyl)-cyclopent-1-enyl]-5(Z)-hept ensäuremethylester:

Die Lösung von 58,3 mg (154 µmol) des nach Beispiel 34 dargestellten Alkohols in 1 ml wasserfreiem Pyridin versetzt man mit 122 mg p-Toluolsulfonsäurechlorid und rührt 1,5 Stunden unter einer Atmosphäre aus trockenem Argon bei 55°C. Man engt ein, versetzt mit Wasser und extrahiert mehrfach mit Dichlormethan. Man wäscht mit Wasser und gesättigter Natriumchloridlösung und trocknet über Magnesiumsulfat. Den nach Filtration und Lösungsmittelabzug erhaltenen Rückstand reinigt man durch Chromatographie an ca. 20 ml Kieselgel mit einem Gradientensystem aus n-Hexan und Ethylacetat. Isoliert werden 61 mg (115 µmol, 74%) der Titelverbindung als farbloses Öl.
IR (Film): 3010, 2950, 2860, 1735, 1600, 1440, 1360, 1190, 1175, 1095, 990, 885, 840, 815, 785, 695 und 665 cm⁻¹.

### Beispiel 37:

### 7-[5R-(3-Methylbenzyloxymethyl)-cyclopent-1-enyl]-5(Z)-heptensäure:

24 mg (67 µmol) der nach Beispiel 36 dargestellten Verbindung verseift man in Analogie zu Beispiel 2 und isoliert nach Aufarbeitung und Reinigung 21,6 mg (62 µmol, 93%) der Titelverbindung als farbloses Öl.
IR (Film): 3600-2400, 3010, 2930, 2860, 1710, 1610, 1590, 1455, 1435, 1360, 1240, 1160, 1105, 1090, 955, 780, 745 und 695 cm⁻¹.

### Beispiel 38:

### 6-[(5S)-(2-Nitrophenylsulfonylaminomethyl)-cyclopent-1-enyl]-4(Z)-hexensäuremethylester:

Die Lösung von 55 mg (max. 125 µmol) des nach Beispiel 38a dargestellten Amins in 0,4 ml wasserfreiem Dichlormethan versetzt man mit 125 µl Triethylamin, 38 mg 2-Nitrobenzolsulfonsäurechlorid und rührt 0,5 Stunden bei 23°C unter einer Atmosphäre aus trockenem Argon. Man gießt auf eine halbkonzentrierte Natriumchloridlösung und extrahiert mehrfach mit Dichlormethan. Die vereinigten organischen Extrakte trocknet man über Magnesiumsulfat und reinigt den nach Lösungsmittelabzug erhaltenen Rückstand durch Chromatographie an 2 analytischen Dünnschichtplatten. Als Laufmittel dient ein Gemisch aus n-Hexan und Ethylacetat, als Elutionsmittel Trichlormethan. Isoliert werden 22 mg (54 µmol, 43%) der Titelverbindung als farbloses Öl.
¹H-NMR (CDCl₃): δ= 1,6-1,75(m,1H), 2-2,4(m,6H), 2,55-2,8(m,3H), 2,97(dd,1H), 3,2-3,3(m,1H), 3,66(s,3H), 5,26(s,1H), 5,32-5,54(m,3H), 7,74(m,2H), 7,87(m,1H), 8,13(m,1H).

### Beispiel 38a:

### 6-[(5S)-(Aminomethyl)-cyclopent-1-enyl]-4(Z)-hexensäuremethylester:

Die Lösung von 392 mg (1,57 mmol) der nach Beispiel 38b dargestellten Verbindung in 13 ml Tetrahydrofuran versetzt man mit 495 mg Triphenylphosphin und rührt 17 Stunden unter einer Atmosphäre aus trockenem Argon bei 23 °C. Anschließend versetzt man mit 2,5 ml Wasser und erhitzt 1 Stunde auf 80°C. Man engt ein, nimmt mit Dichlormethan auf, trocknet über Magnesiumsulfat und reinigt den nach Filtration und Lösungsmittelabzug erhaltenen Rückstand durch Filtration an ca. 100 ml grobem Kieselgel mit einem Gradientensystem aus Dichlormethan, Methanol und Triethylamin. Isoliert werden 621 mg (max. 1,57mmol) noch verunreinigtes Amin, das man ohne zusätzliche Reinigung weiter umsetzt.

### Beispiel 38b:

### 6-[(5S)-(Azidomethyl)-cyclopent-1-enyl]-4(Z)-hexensäuremethylester:

Die Lösung von 523 mg (1,82 mmol) des nach Beispiel 38c dargestellten Bromides in 39 ml Dimethylformamid versetzt man mit 502 mg Natriumazid und erwärmt 2,5 Stunden unter einer Atmosphäre aus trockenem Argon auf 60°C. Man gießt in Eiswasser, extrahiert mehrfach mit Diethylether, trocknet die organische Phase über Magnesiumsulfat und reinigt den nach Filtration und Lösungsmittelabzug erhaltenen Rückstand durch Chromatographie an ca. 70 ml feinem Kieselgel unter Verwendung eines Gradientensystems aus n-Hexan und Ethylacetat. Isoliert werden 392 mg (1,57 mmol, 86%) der Titelverbindung als farbloses Öl.
IR (Film): 2950, 2930, 2850, 2100, 1735, 1435, 1355, 1265 und 1160 cm⁻¹.

### Beispiel 38c:

### 6-[(5S)-(Brommethyl)-cyclopent-1-enyl]-4(Z)-hexensäuremethylester:

Die Lösung von 680 mg (2,78 mmol) der nach Beispiel 38d dargestellten Verbindung B in 20 ml Acetonitril versetzt man mit 1,87 ml Collidin, 4,62 g Tetrabrommethan, 3,65 g Triphenylphosphin und rührt 7 Stunden bei 23°C unter einer Atmosphäre aus trockenem Argon. Man engt ein und reinigt den erhaltenen Rückstand durch Chromatographie an ca. 200 ml feinem Kieselgel unter Verwendung eines Gradientensystems aus n-Hexan und Ethylacetat. Isoliert werden 682 mg (2,37 mmol, 85%) der Titelverbindung als farbloses Öl.
IR (Film): 2950, 2850, 1735, 1440, 1245, 1215 und 1160 cm⁻¹.

### Beispiel 38d:

### 6-[(1R,2S,5R)-2-Hydroxymethyl-5-fluor-cyclopentyl]-4(Z)-hexensäuremethylester (A) und 6-[(5S)-5-hydroxy-cyclopent-1-enyl]-4(Z)-hexensäuremethylester (B):

3,69 g des nach Beispiel 38e dargestellten Substanzgemisches setzt man in Analogie zu Beispiel 1g um und isoliert nach Aufarbeitung und Reinigung 943 mg (4,20 mmol 40%) der Titelverbindung B als unpolare Komponente, sowie 758 mg (3,10 mmol, 30%) der Titelverbindung A als polare Komponente, jeweils als farbloses Öl.
IR (Film) von A: 3200-3600, 3050, 2950, 2870, 1735, 1435, 1360, 1165, 1040, 945 und 875 cm⁻¹.
IR (Film) von B: 3200-3600, 3050, 2940, 2850, 1735, 1435, 1360, 1200, 1160 und 1025 cm⁻¹.

### Beispiel 38e:

### 6-[(1R,2S,5R)-2-tert.-Butyldiphenylsilyloxymethyl-5-fluor-cyclopentyl]-4(Z)-hexensäuremethylester und 6-[(5S)-5-(tert.-Butyldiphenylsilyloxymethyl)-cyclopent-1-enyl]-4(Z)-hexensäuremethylester:

5,0 g (10,4 mmol) der nach Beispiel 38f dargestellten Verbindung setzt man in Analogie zu Beispiel 1 um und isoliert nach Aufarbeitung und Reinigung 3,81 g eines Gemisches der beiden Titelverbindungen als farbloses Öl.

### Beispiel 38f:

### 6-[(1R,2S,5S)-2-tert.-Butyldiphenylsilyloxymethyl-5-hydroxy-cyclopentyl]-4(Z)-hexensäuremethylester:

62,6 g (max. 58,3mol) der nach Beispiel 38g dargestellten Verbindung setzt man in Analogie zu Beispiel 32b um und isoliert nach Aufarbeitung und Reinigung 26,8 g (55,7 mmol, 96%) der Titelverbindung als farbloses Öl.
IR (Film): 3600-3200, 3070, 3040, 2950, 2930, 2850, 1735, 1585, 1460, 1425, 1240, 1160, 1110, 1005, 820, 740 und 700 cm⁻¹.

### Beispiel 38g:

### 6-[(1R,2S,5S)-2-tert.-Butyldiphenylsilyloxymethyl-5-hydroxy-cyclopentyl]-4(Z)-hexensäure:

23,1 g (58,3 mmol) der nach Beispiel 1k dargestellten Verbindung setzt man in Analogie zu Beispiel 1j unter Verwendung von Carboxypropyltriphenylphosphoniumbromid um und isoliert nach Aufarbeitung 62,6 g der Titelverbindung als Rohprodukt, das ohne Reinigung weiter umgesetzt wird.

### Beispiel 39:

### 6-[(5S)-(2-Nitrophenylsulfonylaminomethyl)-cyclopent-1-enyl]-4(Z)-hexensäure:

22 mg (54 µmol) der nach Beispiel 38 dargestellten Verbindung verseift man in Analogie zu Beispiel 2 und isoliert nach Aufarbeitung und Reinigung 18 mg (46 µmol, 85%) der Titelverbindung als farbloses Öl.
¹H-NMR (CDCl₃): δ= 1,55-1,68(m,1H), 1,9-2,4(m,7H), 2,5-2,8(m,3H), 2,92(m,1H), 3,19-(m,1H), 5,19(dd,1H), 5,25-5,5(m,3H), 7,67(m,2H), 7,79(m,1H), 8,06(m,1H).

### Beispiel 40:

### 6-[(5S)-(4-Fluorphenylsulfonylaminomethyl)-cyclopent-1-enyl]-4(Z)-hexensäuremethylester:

55 mg (max. 125 µmol) der nach Beispiel 38a dargestellten Verbindung setzt man in Analogie zu Beispiel 38 unter Verwendung von 4-Fluorbenzolsulfonsäurechlorid um und isoliert nach Aufarbeitung und Reinigung 19 mg (50 µmol, 40%) der Titelverbindung als farbloses Öl.
¹H-NMR (CDCl₃): δ= 1,63-1,75(m,1H), 1,95-2,4(m,7H), 2,5-2,78(m,3H), 2,88(m,1H), 3,1-(m,1H), 4,79(dd,1H), 5,32-5,5(m,3H), 7,19(m,2H), 7,89(m,2H).

### Beispiel 41:

### 6-[(5S)-(4-Fluorphenylsulfonylaminomethyl)-cyclopent-1-enyl]-4(Z)-hexensäure:

19 mg (50 µmol) der nach Beispiel 40 dargestellten Verbindung verseift man in Analogie zu Beispiel 2 und isoliert nach Aufarbeitung und Reinigung 17 mg (46 µmol, 93%) der Titelverbindung als farbloses Öl.
¹H-NMR (CDCl₃): δ= 1,5-1,65(m,1H), 1,87-2,4(m,7H), 2,45-2,72(m,3H), 2,8(m,1H), 3,02-(m,1H), 4,78(dd,1H), 5,3-5,44(m,3H), 7,12(m,2H), 7,8(m,2H).

### Beispiel 42:

### 6-[(5S)-(Phenylsulfonylaminomethyl)-cyclopent-1-enyl]-4(Z)-hexensäuremethylester:

55 mg (max. 125 µmol) der nach Beispiel 38a dargestellten Verbindung setzt man in Analogie zu Beispiel 38 unter Verwendung von Benzolsulfonsäurechlorid um und isoliert nach Aufarbeitung und Reinigung 19 mg (52 µmol, 42%) der Titelverbindung als farbloses Öl.
¹H-NMR (CDCl₃): δ= 1,63-1,75(m,1H), 1,95-2,4(m,6H), 2,48-2,75(m,3H), 2,88(m,1H), 3,12(m,1H), 3,68(s,3H), 4,69(dd,1H), 5,32-5,5(m,3H), 7,48-7,62(m,3H), 7,88(m,2H).

### Beispiel 43:

### 6-[(5S)-(Phenylsulfonylaminomethyl)-cyclopent-1-enyl]-4(Z)-hexensäure:

19 mg (52 µmol) der nach Beispiel 42 dargestellten Verbindung verseift man in Analogie zu Beispiel 2 und isoliert nach Aufarbeitung und Reinigung 16 mg (46 µmol, 88%) der Titelverbindung als farbloses Öl.
¹H-NMR (CDCl₃): δ= 1,52-1,64(m,1H), 1,87-2,7(m,9H), 2,8(m,1H), 3,02(m,1H), 4,72(dd,-1H), 5,25-5,45(m,3H), 7,4-7,56(m,3H), 7,8(m,2H).

### Beispiel 44:

### 6-[(5S)-(4-Methylphenylsulfonylaminomethyl)-cyclopent-1-enyl]-4(Z)-hexensäuremethylester:

55 mg (max. 125 µmol) der nach Beispiel 38a dargestellten Verbindung setzt man in Analogie zu Beispiel 38 unter Verwendung von 4-Toluolsulfonsäurechlorid um und isoliert nach Aufarbeitung und Reinigung 20 mg (53 µmol, 42%) der Titelverbindung als farbloses Öl.
¹H-NMR (CDCl₃): δ= 1,66-1,79(m,1H), 2-2,45(m,6H), 2,49-2,8(m,3H), 2,91(m,1H), 3,12-(m,1H), 3,68(s,3H), 5,07(dd,1H), 5,35-5,5(m,3H), 7,6(d,1H), 7,7(dd,1H), 7,98(d,1H).

### Beispiel 45:

### 6-[(5S)-(4-Methylphenylsulfonylaminomethyl)-cyclopent-1-enyl]-4(Z)-hexensäure:

20 mg (53 µmol) der nach Beispiel 44 dargestellten Verbindung verseift man in Analogie zu Beispiel 2 und isoliert nach Aufarbeitung und Reinigung 18 mg (50 µmol, 93%) der Titelverbindung als farbloses Öl.
¹H-NMR (CDCl₃): δ= 1,5-1,63(m,1H), 1,86-2,4(m,10H), 2,45-2,7(m,3H), 2,76(m,1H), 3(m,1H), 3,67(s,3H), 4,67(dd,1H), 5,27-5,42(m,3H), 7,22(d,2H), 7,68(d,2H).

### Beispiel 46:

### 6-[(5S)-(3,4-Dichlorphenylsulfonylaminomethyl)-cyclopent-1-enyl]-4(Z)-hexensäuremethylester:

55 mg (max. 125 µmol) der nach Beispiel 38a dargestellten Verbindung setzt man in Analogie zu Beispiel 38 unter Verwendung von 3,4-Dichlorbenzolsulfonsäurechlorid um und isoliert nach Aufarbeitung und Reinigung 21 mg (49 µmol, 39%) der Titelverbindung als farbloses Öl.
¹H-NMR (CDCl₃): δ= 1,65-1,78(m,1H), 2-2,45(m,7H), 2,48-2,82(m,3H), 2,92(m,1H), 3,12-(m,1H), 5,08(dd,1H), 5,35-5,5(m,3H), 7,6(d,1H), 7,7(dd,2H), 7,98(d,1H).

### Beispiel 47:

### 6-[(5S)-(3,4-Dichlorphenylsulfonylaminomethyl)-cyclopent-1-enyl]-4(Z)-hexensäure:

21 mg (75 µmol) der nach Beispiel 46 dargestellten Verbindung verseift man in Analogie zu Beispiel 2 und isoliert nach Aufarbeitung und Reinigung 15 mg (36 µmol, 48%) der Titelverbindung als farbloses Öl.
¹H-NMR (CDCl₃): δ= 1,55-1,68(m,1H), 1,92-2,4(m,8H), 2,45-2,75(m,3H), 2,82(m,1H), 3,04(m,1H), 4,94(dd,1H), 5,3-5,45(m,3H), 7,53(d,1H), 7,62(dd,1H), 7,9(d,1H).

### Beispiel 48:

### 6-[(5S)-(4-Chlorphenylsulfonylaminomethyl)-cyclopent-1-enyl]-4(Z)-hexensäuremethylester:

55 mg (max. 125 µmol) der nach Beispiel 38a dargestellten Verbindung setzt man in Analogie zu Beispiel 38 unter Verwendung von 4-Chlorbenzolsulfonsäurechlorid um und isoliert nach Aufarbeitung und Reinigung 25 mg (63 µmol, 50%) der Titelverbindung als farbloses Öl.
¹H-NMR (CDCl₃): δ= 1,62-1,74(m,1H), 1,96-2,4(m,7H), 2,5-2,77(m,3H), 2,88(m,1H), 3,08(m,1H), 3,67(s,3H), 4,8(dd,1H), 5,35-5,5(m,3H), 7,49(d,2H), 7,82(d,2H).

### Beispiel 49:

### 6-[(5S)-(4-Chlorphenylsulfonylaminomethyl)-cyclopent-1-enyl]-4(Z)-hexensäure:

25 mg (63 µmol) der nach Beispiel 48 dargestellten Verbindung verseift man in Analogie zu Beispiel 2 und isoliert nach Aufarbeitung und Reinigung 18 mg (47 µmol, 74%) der Titelverbindung als farbloses Öl.
¹H-NMR (CDCl₃): δ= 1,58-1,7(m,1H), 1,95-2,45(m,7H), 2,53-2,78(m,3H), 2,86(m,1H), 3,08(m,1H), 4,84(dd,1H), 5,35-5,5(m,3H), 7,49(d,2H), 7,8(d,2H).

### Beispiel 50:

### 7-[(5S)-(2,4-Difluorphenylsulfonylaminomethyl)-cyclopent-1-enyl]-5(Z)-heptensäuremethylester:

143 mg (max. 246 µmol) der nach Beispiel 50a dargestellten Verbindung setzt man in Analogie zu Beispiel 38 unter Verwendung von 2,4-Difluorbenzolsulfonsäurechlorid um und isoliert nach Aufarbeitung und Reinigung 12 mg (29 µmol, 12%) der Titelverbindung als farbloses Öl.
IR (Film): 3200-3350, 3010, 2950, 2850, 1730, 1590, 1490, 1435, 1330, 1235, 1170, 1150, 1095, 840 und 670 cm⁻¹.

### Beispiel 50a:

### 7-[(5S)-(Aminomethyl)-cyclopent-1-enyl]-5(Z)-heptensäuremethylester:

Die Lösung von 765 mg (3,07 mmol) der nach Beispiel 50b dargestellten Verbindung in 26 ml Tetrahydrofuran versetzt man mit 969 mg Triphenylphosphin und erwärmt 3,5 Stunden unter einer Atmosphäre aus trockenem Argon auf 50°C. Anschließend versetzt man mit 3,3 ml Wasser und erhitzt 1 Stunde zum Rückfluß. Man engt ein, nimmt mit Dichlormethan auf, trocknet über Magnesiumsulfat und isoliert nach Filtration und Lösungsmittelabzug 1,79 g (max. 3,07 mmol) mit Triphenylphosphin und Triphenylphosphinoxid verunreinigtes Amin, das man ohne Reinigung weiter umsetzt.

### Beispiel 50b:

### 7-[(5S)-(Azidomethyl)-cyclopent-1-enyl]-5(Z)-heptensäuremethylester:

Die Lösung von 933 mg (3,09 mmol) des nach Beispiel 50c dargestellten Bromides in 67 ml Dimethylformamid versetzt man mit 853 mg Natriumazid und erwärmt 3 Stunden unter einer Atmosphäre aus trockenem Argon auf 60°C. Man gießt in Eiswasser, extrahiert mehrfach mit Diethylether, trocknet die organische Phase über Magnesiumsulfat und reinigt den nach Filtration und Lösungsmittelabzug erhaltenen Rückstand durch Chromatographie an ca. 200 ml feinem Kieselgel unter Verwendung eines Gradientensystems aus n-Hexan und Ethylacetat. Isoliert werden 765 mg (2,90 mmol, 94%) der Titelverbindung als farbloses Öl.
IR (Film): 2950, 2850, 2100, 1735, 1435, 1355, 1260 und 1160 cm⁻¹.

### Beispiel 50c:

### 7-[(5S)-(Brommethyl)-cyclopent-1-enyl]-5(Z)-heptensäuremethylester:

Die Lösung von 941 mg (3,95 mmol) 7-[(5S)-5-Hydroxy-cyclopent-1-enyl]-5(Z)-heptensäuremethylester, der in Analogie zu den Beispielen 38d bis 38g und 1j unter Verwendung von Carboxybutyltriphenylphosphoniumbromid dargestellt wird, in 29 ml Acetonitril versetzt man mit 2,66 ml Collidin, 6,56 g Tetrabrommethan, 5,18 g Triphenylphosphin und rührt 6 Stunden bei 23°C unter einer Atmosphäre aus trockenem Argon. Man engt ein und reinigt den erhaltenen Rückstand durch Chromatographie an ca. 200 ml feinem Kieselgel unter Verwendung eines Gradientensystems aus n-Hexan und Ethylacetat. Isoliert werden 933 mg (3,25 mmol, 82%) der Titelverbindung als farbloses Öl.
IR (Film): 2950, 2850, 1735, 1435, 1245, 1215 und 1160 cm⁻¹.

### Beispiel 51:

### 7-[(5S)-(2,4-Difluorphenylsulfonylaminomethyl)-cyclopent-1-enyl]-5(Z)-heptensäure:

12 mg (30 µmol) der nach Beispiel 50 dargestellten Verbindung verseift man in Analogie zu Beispiel 2 und isoliert nach Aufarbeitung und Reinigung 4 mg (10 µmol, 33%) der Titelverbindung als farbloses Öl.
¹H-NMR (CDCl₃): δ= 1,6-1,75(m,3H), 1,95-2,4(m,8H), 2,5-2,8(m,3H), 2,97(m,1H), 3,13-(m,1H), 5,03(dd,1H), 5,37-5,54(m,3H), 6,93-7,05(m,2H), 7,92(m,1H).

### Beispiel 52:

### 7-[(5S)-(3,4-Dimethoxyphenylsulfonylaminomethyl)-cyclopent-1-enyl]-5(Z)-heptensäuremethylester:

144 mg (max. 246 µmol) der nach Beispiel 50a dargestellten Verbindung setzt man in Analogie zu Beispiel 38 unter Verwendung von 3,4-Dimethoxybenzolsulfonsäurechlorid um und isoliert nach Aufarbeitung und Reinigung 14 mg (32 µmol, 13%) der Titelverbindung als farbloses Öl.
¹H-NMR (CDCl₃): δ= 1,5-1,72(m,3H), 1,85-2,5(m,8H), 2,56-2,74(m,2H), 2,86(m,1H), 2,98(m,1H), 3,65(s,3H), 3,83(s,3H), 3,87(s,3H), 4,8(dd,1H), 5,28-5,45(m,3H), 6,86(d,1H), 7,29(d,1H), 7,4(dd,1H)

### Beispiel 53:

### 7-[(5S)-(3,4-Dimethoxyphenylsulfonylaminomethyl)-cyclopent-1-enyl]-5(Z)-heptensäure:

11 mg (34 µmol) der nach Beispiel 52 dargestellten Verbindung verseift man in Analogie zu Beispiel 2 und isoliert nach Aufarbeitung und Reinigung 8 mg (19 µmol, 56%) der Titelverbindung als farbloses Öl.
¹H-NMR (CDCl₃): δ= 1,49-1,7(m,3H), 1,87-2,48(m,8H), 2,59-2,73(m,2H), 2,85(m,1H), 2,98(m,1H), 3,85(s,3H), 3,88(s,3H), 4,8(dd,1H), 5,28-5,45(m,3H), 6,87(d,1H), 7,3(d,1H), 7,42(dd,1H)

### Beispiel 54:

### 7-[(5S)-(4-Fluorphenylsulfonylaminomethyl)-cyclopent-1-enyl]-5(Z)-heptensäuremethylester:

143 mg (max. 246 µmol) der nach Beispiel 50a dargestellten Verbindung setzt man in Analogie zu Beispiel 38 unter Verwendung von 4-Fluorbenzolsulfonsäurechlorid um und isoliert nach Aufarbeitung und Reinigung 35 mg (88 µmol, 36%) der Titelverbindung als farbloses Öl.
IR (Film): 3200-3400, 3010, 2940, 2850, 1730, 1590, 1490, 1435, 1330, 1235, 1175, 1150, 1090, 840 und 670 cm⁻¹.

### Beispiel 55:

### 7-[(5S)-(4-Fluorphenylsulfonylaminomethyl)-cyclopent-1-enyl]-5(Z)-heptensäure:

28 mg (73 µmol) der nach Beispiel 54 dargestellten Verbindung verseift man in Analogie zu Beispiel 2 und isoliert nach Aufarbeitung und Reinigung 14 mg (37 µmol, 50%) der Titelverbindung als farbloses Öl.
¹H-NMR (CDCl₃): δ= 1,5-1,7(m,3H), 1,85-2,52(m,11H), 2,55-2,7(m,2H), 2,85(m,1H), 3(m,1H), 5(m,1H), 5,25-5,45(m,3H), 7,12(m,2H), 7,82(m,2H).

### Beispiel 56:

### 7-[(5S)-(2-Nitrophenylsulfonylaminomethyl)-cyclopent-1-enyl]-5(Z)-heptensäuremethylester:

143 mg (max. 246 µmol) der nach Beispiel 50a dargestellten Verbindung setzt man in Analogie zu Beispiel 38 unter Verwendung von 2-Nitrobenzolsulfonsäurechlorid um und isoliert nach Aufarbeitung und Reinigung 32 mg (76 µmol, 31%) der Titelverbindung als farbloses Öl.
IR (Film): 3200-3400, 3090, 3010, 2940, 2850, 1730, 1590, 1540, 1435, 1410, 1360, 1340, 1170, 850, 780, 740, 730 und 655 cm⁻¹.

### Beispiel 57:

### 7-[(5S)-(2-Nitrophenylsulfonylaminomethyl)-cyclopent-1-enyl]-5(Z)-heptensäure:

32 mg (76 µmol) der nach Beispiel 56 dargestellten Verbindung verseift man in Analogie zu Beispiel 2 und isoliert nach Aufarbeitung und Reinigung 19 mg (47 µmol, 61%) der Titelverbindung als farbloses Öl.
¹H-NMR (CDCl₃): δ= 1,6-1,75(m,3H), 1,95-2,4(m,7H), 2,53-2,83(m,3H), 2,99(m,1H), 3,24(m,1H), 5,26(dd,1H), 5,33-5,55(m,3H), 7,75(m,2H), 7,87(m,1H),8,12(m,1H).

### Beispiel 58:

### 7-[(5S)-(3,4-Dichlorphenylsulfonylaminomethyl)-cyclopent-1-enyl]-5(Z)-heptensäuremethylester:

143 mg (max. 246 µmol) der nach Beispiel 50a dargestellten Verbindung setzt man in Analogie zu Beispiel 38 unter Verwendung von 3,4-Dichlorbenzolsolfonsäurechlorid um und isoliert nach Aufarbeitung und Reinigung 34 mg (76 µmol, 31%) der Titelverbindung als farbloses Öl.
IR (Film): 3200-3350, 3010, 2940, 2850, 1730, 1450, 1370, 1335, 1160, 1030, 820 und 675 cm⁻¹.

### Beispiel 59:

### 7-[(5S)-(3,4-Dichlorphenylsulfonylaminomethyl)-cyclopent-1-enyl]-5(Z)-heptensäure:

34 mg (76 µmol) der nach Beispiel 58 dargestellten Verbindung verseift man in Analogie zu Beispiel 2 und isoliert nach Aufarbeitung und Reinigung 19 mg (44 µmol, 58%) der Titelverbindung als farbloses Öl.
¹H-NMR (CDCl₃): δ= 1,5-1,72(m,3H), 1,88-2,5(m,9H), 2,58-2,73(m,2H), 2,88(m,1H), 3,03(m,1H), 5,02(dd,1H), 5,27-5,45(m,3H), 7,52(d,1H), 7,63(dd,1H), 7,9(d,1H).

### Beispiel 60:

### 7-[(5S)-(Phenylsulfonylaminomethyl)-cyclopent-1-enyl]-5(Z)-heptensäuremethylester:

143 mg (max. 246 µmol) der nach Beispiel 50a dargestellten Verbindung setzt man in Analogie zu Beispiel 38 unter Verwendung von Benzolsulfonsäurechlorid um und isoliert nach Aufarbeitung und Reinigung 31 mg (85 µmol, %) der Titelverbindung als farbloses Öl.
IR (Film): 3200-3350, 3010, 2940, 2850, 1730, 1445, 1325, 1160, 1090, 755, 720 und 690 cm⁻¹.

### Beispiel 61:

### 7-[(5S)-(Phenylsulfonylaminomethyl)-cyclopent-1-enyl]-5(Z)-heptensäure:

31 mg (85 µmol) der nach Beispiel 60 dargestellten Verbindung verseift man in Analogie zu Beispiel 2 und isoliert nach Aufarbeitung und Reinigung 20 mg (55 µµmol, 65%) der Titelverbindung als farbloses Öl.
¹H-NMR (CDCl₃): δ= 1,45-1,7(m,3H), 1,85-2,5(m,8H), 2,57-2,7(m,2H), 2,83(m,1H), 3,01-(m,1H), 4,83(dd,1H), 5,26-5,45(m,3H), 7,4-7,57(m,3H), 7,8(m,2H).

## Patentansprüche

1. Cyclopentenderivate der Formel I, 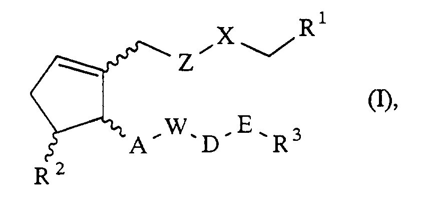 worin R¹ 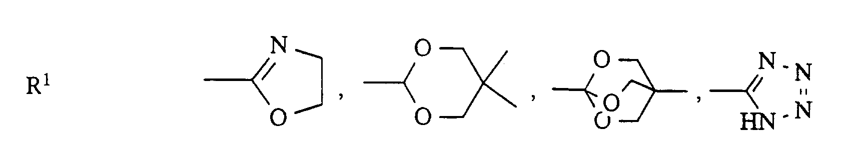 oder COOR⁴, wobei R⁴ Wasserstoff oder gegebenenfalls durch Halogen, Phenyl, C₁-C₄-Alkoxy oder Di-(C₁-C₄)-alkylamino substituiertes C₁-C₁₀-Alkyl, C₃-C₁₀-Cycloalkyl, C₇-C₁₆-Aralkyl, durch Y sub-stituiertes Phenacyl oder C₆-C₁₂-Aryl oder einen 5- oder 6- gliedrigen heterocyclischen Rest mit wenigstens einem N-, O- oder S-Atom bedeuten kann, oder -CONHR⁶ mit R⁶ in der Bedeutung Wasserstoff, C₁-C₁₀-Alkyl, C₁-C₁₀-Alkanoyl,C₆-C₁₂-Arylsulfonyl oder C₁-C₁₀-Alkansulfonyl sein kann,
X -(CH₂)ₚ-, -CH₂-O- oder -CH₂-S-,
Z,A unabhängig voneinander eine Direktbindung, (Z)-CH=CH-, (E)-CH=CH- oder -C≡C-,
p 0 bis 5,
n,r unabhängig voneinander 0 bis 2,
R² OR⁵ oder R⁵,
W eine Direktbindung, eine -[(CH₂)ₙ-V]_{q}-Guppe, eine -(CH₂)ₙ-V-(CH₂)_{q}-V-Gruppe, eine freie oder funktionell abgewandelte Hydroxymethylengruppe oder eine freie oder funktionell abgewandelte 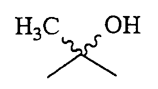 -Gruppe, wobei die Hydroxygruppe jeweils α- oder β-ständig sein kann,
q 1 oder 2,
D eine Direktbindung, eine geradkettige gesättigte Alkylengruppe mit 1-5 C-Atomen, eine verzweigte gesättigte Alkylengruppe oder eine geradkettige oder verzweigte ungesättigte Alkylengruppe mit 2-5 C-Atomen, die gegebenenfalls durch Fluoratome substituiert sein können, -(CH₂)ₙ-NH-SO₂-, 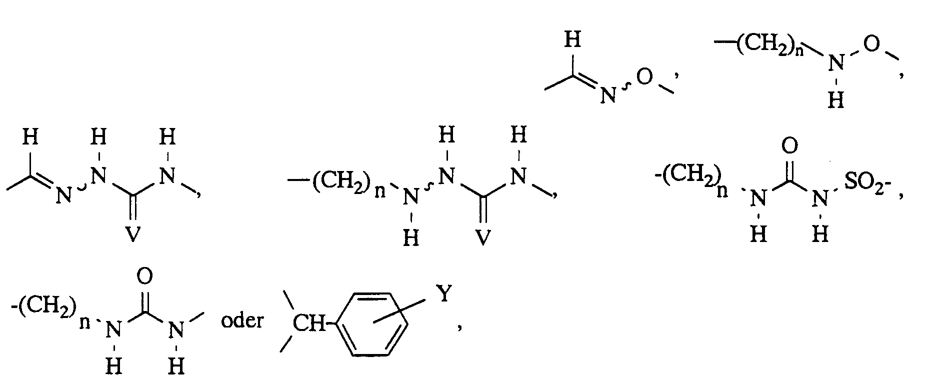 V ein O- oder S-Atom,
E eine Direktbindung, -C≡C- oder -CH=CR⁷-, wobei R⁷ Wasserstoff, C₁-C₅-Alkyl, Halogen oder Trifluormethyl,
AWDE gemeinsam eine Direktbindung,
AW gemeinsam eine Direktbindung,
DE gemeinsam eine Direktbindung sein können, 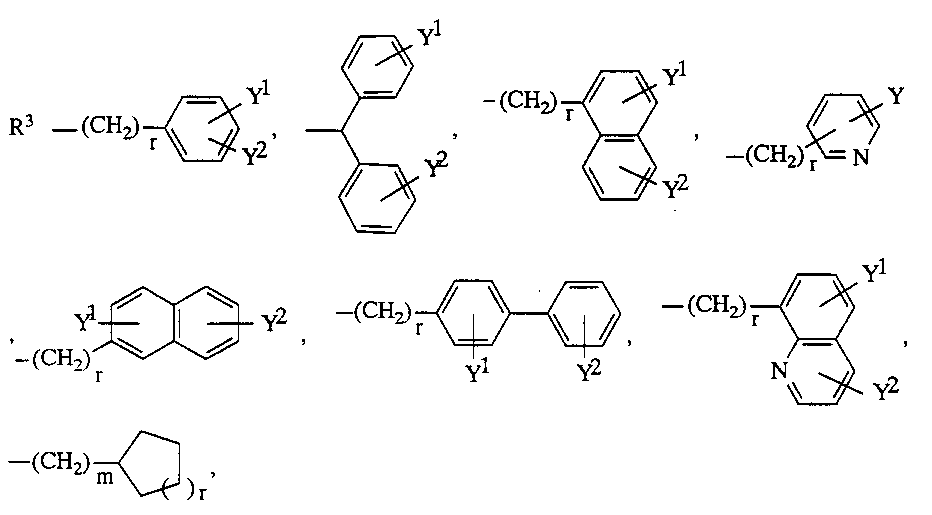 C₃-C₁₀-Cycloalkyl oder gegebenenfalls durch Y substituiertes C₁-C₁₀-Alkyl,
wobei R³ nur 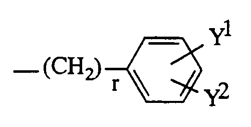 sein kann, wenn A oder W eine Direktbindung bedeuten,
m 1 oder 2,
Y¹ und Y² gleich oder verschieden sind und Y bedeuten,
Y Wasserstoff, Halogen, N₃, CF₃, OR⁵, NO₂, NH₂, CN, COOR⁵ oder C₁-C₁₀-Alkyl,
R⁵ Wasserstoff, gegebenenfalls durch Halogen substituiertes C₁-C₁₀-Alkyl, C₆-C₁₂-Aryl oder C₇-C₁₆-Aralkyl sein kann und, falls R⁴ Wasserstoff bedeutet, deren Salze mit physiologisch verträglichen Basen, sowie die α-, β- oder γ-Cyclodextrinclathrate, sowie die mit Liposomen verkapselten Verbindungen der Formel I bedeuten.

2. Arzneimittel bestehend aus einer oder mehreren Verbindungen des Anspruchs 1 und üblichen Hilfs-, Träger- und Zusatzstoffen.

3. Verfahren zur Herstellung von Cyclopenten-Derivaten der Formel I, dadurch gekennzeichnet, daß man die Hydroxy-Verbindung der Formel II 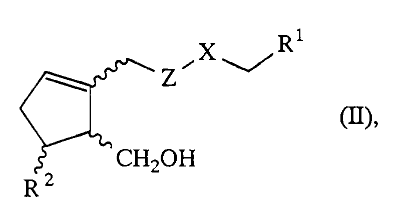 worin R¹, R², X und Z die oben angegebenen Bedeutungen haben und R¹ eine -COOR⁴-Estergruppe mit R⁴ in der mit Ausnahme von Wasserstoff oben angegebenen Bedeutung darstellt, nach Oxidation mit Oxalylchlorid/DMSO mit einem Dimethylphosphonat der Formel V 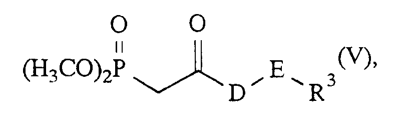 worin D, E und R³ die oben genannte Bedeutung haben, in Gegenwart von Natriumhydrid oder Natriumhydrid/Brom umsetzt, anschließend reduziert und gegebenenfalls Bromwasserstoff abspaltet oder nach Bromierung, Azidbildung und Reduktion das intermediäre Amin der Formel VI 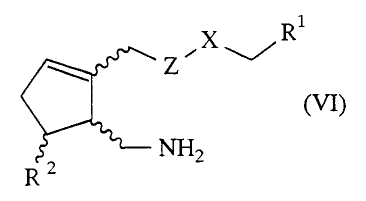 mit einer Verbindung der Formel Hal-SO₂-R³ (VII) oder O=C=N-R³ (VIII), worin Hal und R³ die oben genannte Bedeutung haben, umsetzt oder eine Verbindung der Formel IX oder X fluoriert, 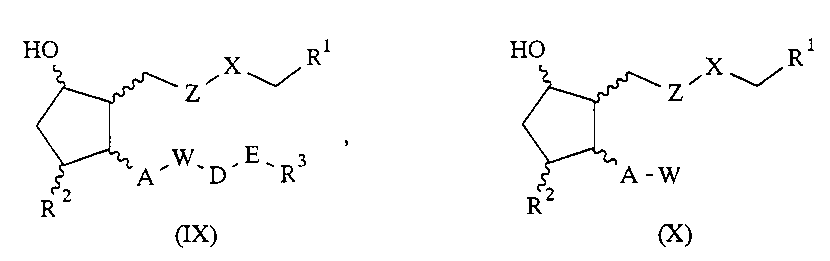 gegebenenfalls mit einer Verbindung der Formel Hal-R³ (XII), worin Hal und R³ die oben genannte Bedeutung haben umsetzt, gegebenenfalls C-C-Mehrfachbindungen hydriert und die erhaltenen Ester verseift, in Salze überführt, in Cyclodextrinclathrate umwandelt oder mit Liposomen verkapselt.

## Claims

1. Cyclopentene derivatives of the formula I 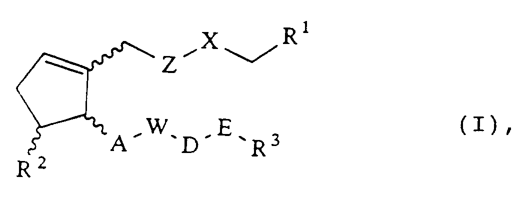 wherein
R¹ can be 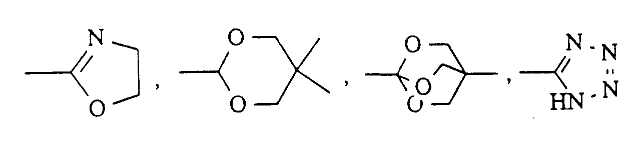 or COOR⁴,
wherein R⁴ can represent hydrogen or optionally halo-, phenyl-, C₁-C₄alkoxy- or di(C₁-C₄alkyl)amino-substituted C₁-C₁₀alkyl, C₃-C₁₀cycloalkyl or C₇-C₁₆aralkyl, Y-substituted phenacyl or C₆-C₁₂aryl, or a 5- or 6-membered heterocyclic radical having at least one N, O or S atom, or R¹ can be -CONHR⁶, in which R⁶ represents hydrogen, C₁-C₁₀alkyl, C₁-C₁₀alkanoyl, C₆-C₁₂arylsulfonyl or C₁-C₁₀alkanesulfonyl,
X represents -(CH₂)ₚ-, -CH₂-O- or -CH₂-S-,
Z and A, each independently of the other, represent a direct bond, (Z)-CH=CH-, (E)-CH=CH- or -C≡C-,
p is from 0 to 5,
n and r, each independently of the other, are from 0 to 2,
R² represents OR⁵ or R⁵,
W represents a direct bond, a group -[(CH₂)ₙ-V]_{q}-, a group -(CH₂)ₙ-V-(CH₂)_{q}-V-, a free or functionally modified hydroxymethylene group or a free or functionally modified group 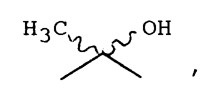 it being possible for the hydroxy group in each case to be in the α- or β-configuration,
q is 1 or 2,
D represents a direct bond, a straight-chained, saturated alkylene group having from 1 to 5 carbon atoms, a branched, saturated alkylene group or a straight-chained or branched, unsaturated alkylene group having from 2 to 5 carbon atoms, each of which may optionally be substituted by fluorine atoms, -(CH₂)ₙ-NH-SO₂-, 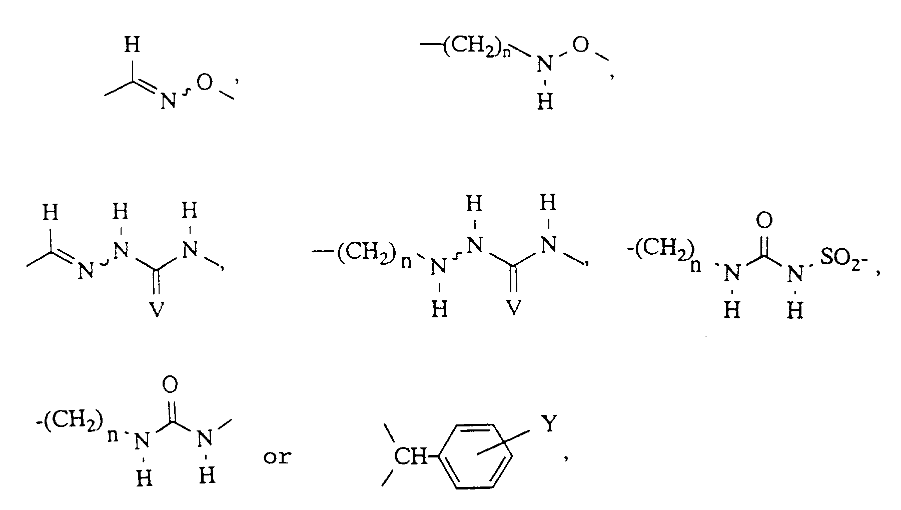 V represents an O or S atom,
E represents a direct bond, -C≡C- or -CH=CR⁷-, wherein R⁷ can be hydrogen, C₁-C₅alkyl, halogen or trifluoromethyl,
AWDE together can be a direct bond,
AW together can be a direct bond, and
DE together can be a direct bond,
R³ represents 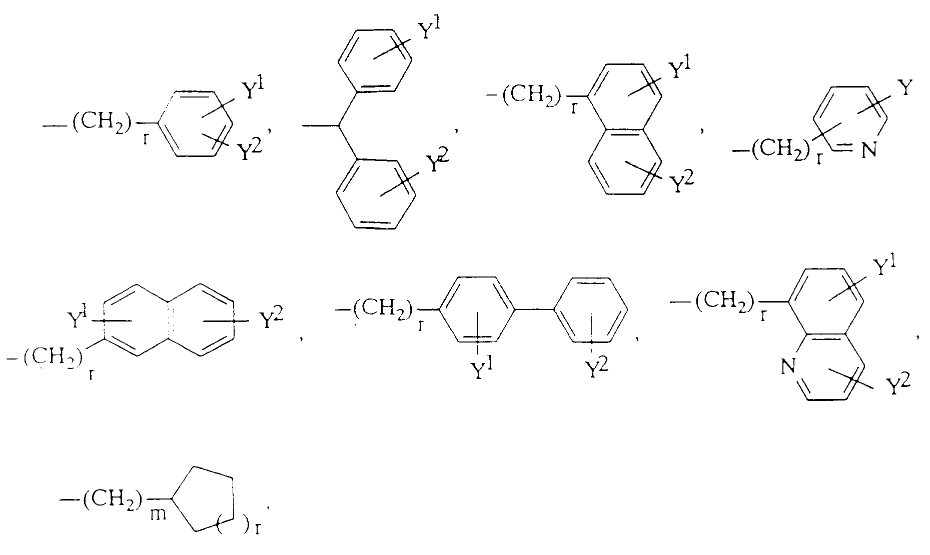 C₃-C₁₀cycloalkyl or optionally Y-substituted C₁-C₁₀alkyl,
wherein R³ can be 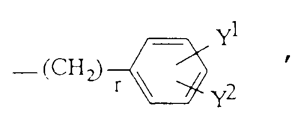 only if A or W represents a direct bond,
m is 1 or 2,
Y¹ and Y² are identical or different and represent Y,
Y represents hydrogen, halogen, N₃, CF₃, OR⁵, NO₂, NH₂, CN, COOR⁵ or C₁-C₁₀alkyl,
R⁵ can be hydrogen or optionally halo-substituted C₁-C₁₀alkyl, C₆-C₁₂aryl or C₇-C₁₆aralkyl,
and, when R⁴ represents hydrogen, the salts thereof with physiologically tolerable bases, as well as α-, β- or - cyclodextrin clathrates, as well as the compounds of the formula I encapsulated with liposomes.

2. Medicaments consisting of one or more compounds of claim 1 and customary excipients, carriers and additives.

3. Process for the preparation of cyclopentene derivatives of the formula I, characterised in that the hydroxy compound of the formula II 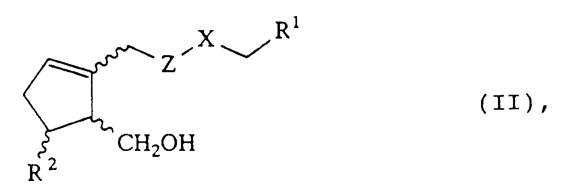 wherein R¹, R², X and Z have the meanings given above and R¹ represents a -COOR⁴ ester group, in which R⁴ has the meaning given above with the exception of hydrogen, is reacted, after oxidation with oxalyl chloride/dimethyl sulphoxide, in the presence of sodium hydride/bromine with a dimethyl phosphonate of the formula V 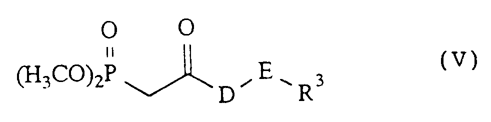 in which D, E and R³ have the meaning given above, is then reduced and, optionally, hydrogen bromide is removed or, after bromination, azide formation and reduction, the intermediate amine of the formula VI 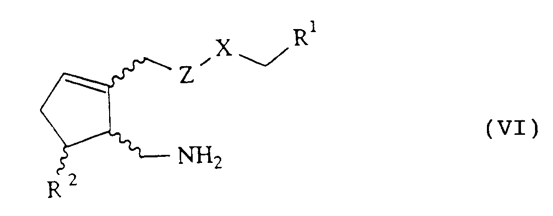 is reacted with a compound of the formula Hal-SO₂-R³ (VII) or O=C=N-R³ (VIII), in which Hal and R³ have the meanings given above, or a compound of the formula IX or X, 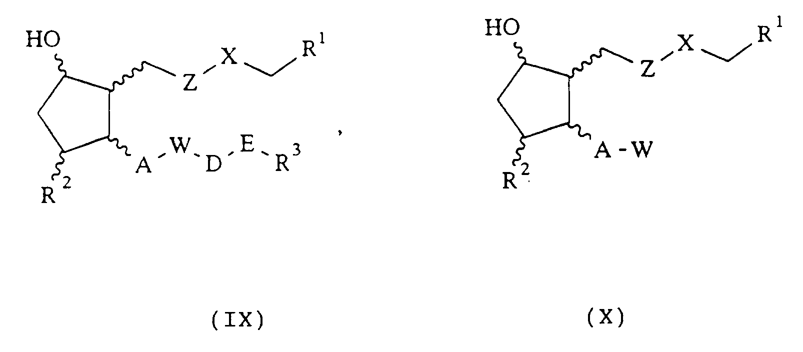 is fluorinated, optionally reacted with a compound of the formula Hal-R³ (XII), in which Hal and R³ have the meanings given above, C-C multiple bonds are optionally hydrogenated and the esters obtained are hydrolysed, converted into salts, converted into cyclodextrin clathrates or encapsulated with liposomes.

## Revendications

1. Dérives de cyclopentène de formule I : 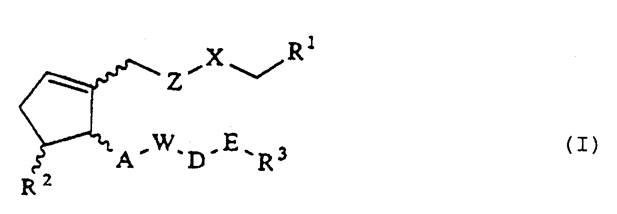 dans laquelle
signifie R¹ 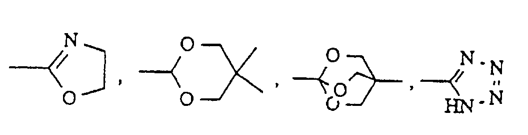 ou COOR⁴, R⁴ pouvant signifier un hydrogène ou un aralkyle en C₇-C₁₆, un cycloalkyle en C₃-C₁₀, un alkyle en C₁-C₁₀ éventuellement substitué par des halogène, phényle, alcoxy en C₁-C₄ ou di(alkyl en C₁-C₄)-amino, un aryle en C₆-C₁₂ ou un phénacyle substitué par Y, ou bien un reste hétérocyclique à 5 ou 6 chaînons ayant au moins un atome N, O ou S, ou bien -CONHR⁶ avec R⁶ pouvant être dans la signification d'un hydrogène, d'un alkyle en C₁-C₁₀, d'un alcanoyle en C₁-C₁₀, d'un arylsulfonyle en C₆-C₁₂ ou d'un alcanesulfonyle en C₁-C₁₀,
X signifie -(CH₂)ₚ-, -CH₂O, ou -CH₂-S-,
Z, A signifient, indépendamment l'un de l'autre, une liaison directe, (Z)-CH=CH-, (E)-CH=CH-, ou -C≡C-,
p vaut de 0 à 5,
n, r valent, indépendamment l'un de l'autre, de 0 à 2,
R² signifie OR⁵ ou R⁵,
W signifie une liaison directe, un groupe -[(CH₂)ₙ-V]_{q}-, un groupe -(CH₂)ₙ-V-(CH₂)_{q}-V-, un groupe hydroxyméthylène libre ou modifié fonctionnellement, ou un groupe 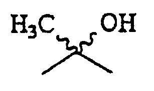 libre ou modifié fonctionnellement, le groupe hydroxy pouvant être à chaque fois en position α ou β,
q vaut 1 ou 2,
D signifie une liaison directe, un groupe alkylène à chaîne droite saturée ayant de 1 à 5 atomes de carbone, un groupe alkylène ramifié saturé ou un groupe alkylène, à chaîne droite ou ramifiée, insaturé, ayant de 2 à 5 atomes de carbone, qui peuvent éventuellement être substitués par des atomes de fluor,
-(CH₂)ₙ-NH-SO₂-, 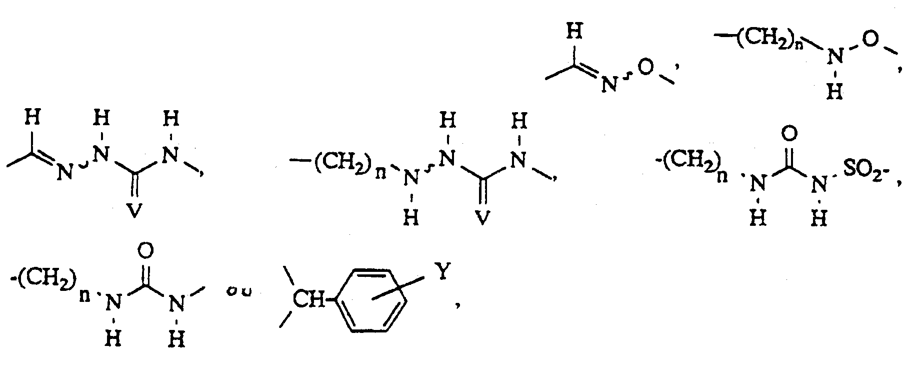
V signifie un atome O ou S,
E signifie une liaison directe, un -C≡C- ou un -CH=CR⁷-,
R⁷ pouvant être un hydrogène, un alkyle en C₁-C₅, un halogène ou un trifluorométhyle,
AWDE peuvent être ensemble une liaison directe,
AW peuvent être ensemble une liaison directe,
DE peuvent être ensemble une liaison directe,
R³ signifie 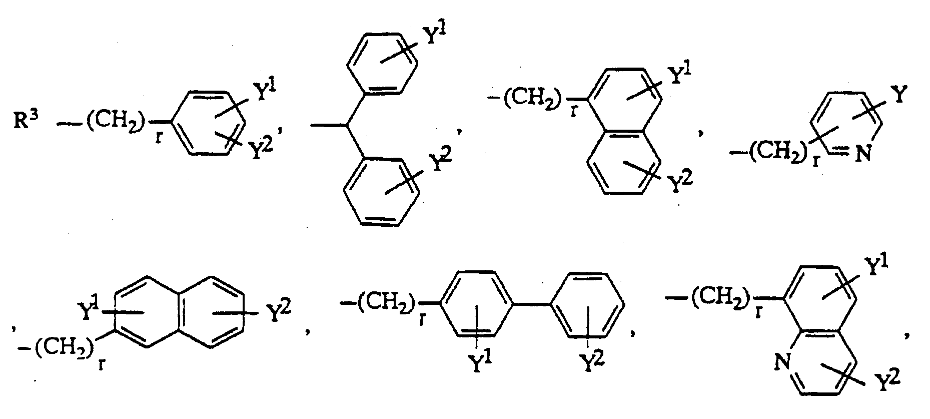 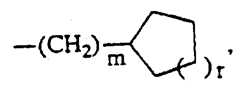 un cycloalkyle en C₃-C₁₀ ou un alkyle en C₁-C₁₀ éventuellement substitué par Y,
R³ pouvant être seulement 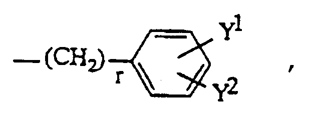 lorsque A ou W signifient une liaison directe,
m vaut 1 ou 2,
Y¹ et Y² sont identiques ou différents et signifient Y,
Y signifie un hydrogène, un halogène, un N₃, un CF₃, un OR⁵, un NO₂, un NH₂, un CN, un COOR⁵ ou un alkyle en C₁-C₁₀,
R⁵ peut être un hydrogène, un aralkyle en C₇-C₁₆, un aryle en C₆-C₁₂ ou un alkyle en C₁-C₁₀ éventuellement substitué par des halogènes et l'invention concerne, lorsque R⁴ signifie l'hydrogène, leurs sels avec des bases physiologiquement compatibles, ainsi que les clathrates d'α-, de β- ou de γ-cyclodextrine, ainsi que les composés de formule I encapsulés avec des liposomes.

2. Médicaments consistant en un ou plusieurs composés selon la revendication 1 et en substances auxiliaires, substances vecteur et additifs classiques.

3. Procédé de préparation de dérivés du cyclopentène de formule I, caractérisé en ce qu'on met en réaction le composé hydroxy de formule II : 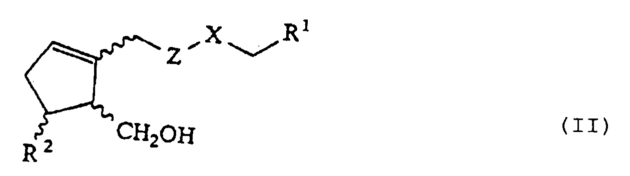 dans laquelle R¹, R², X et Z ont les significations données ci-dessus et R¹ représente un groupe ester - COOR⁴ avec R⁴ ayant la signification donnée ci-dessus à l'exception de l'hydrogène, après oxydation avec le système chlorure d'oxalyle/DMSO, avec un diméthylphosphonate de formule V : 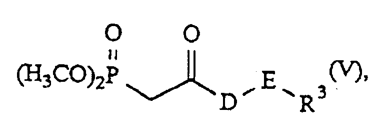 dans laquelle D, E et R³ ont la signification donnée ci-dessus, en présence d'hydrure de sodium ou du système d'hydrure de sodium/brome, ensuite on réalise une réduction et éventuellement une élimination de bromure d'hydrogène ou bien on met en réaction, après bromation, formation de l'azidure et réduction, l'amine intermédiaire de formule VI : 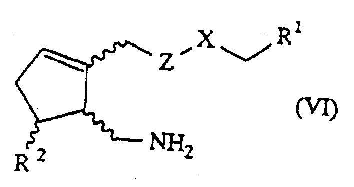 avec un composé de formule Hal-SO₂-R³ (VII) ou O=C=N-R³ (VIII), dans lesquelles Hal et R³ ont la signification donnée ci-dessus, ou bien on réalise une fluoration sur un composé de formule IX ou X, 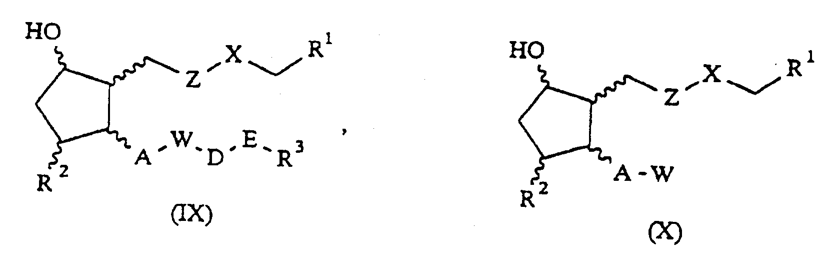 puis on réalise une réaction éventuellement avec un composé de formule Hal-R³ (XII), dans laquelle Hal et R³ ont la signification donnée ci-dessus, on hydrogène éventuellement les liaisons multiples carbone-carbone et on saponifie l'ester obtenu que l'on convertit en sel, que l'on transforme en clathrate de cyclodextrine ou que l'on encapsule avec des liposomes.
